# EUROPEAN PATENT APPLICATION

(11) **EP 4 772 128 A2**
(43) Date of publication of application: **08.07.2026**
(21) Application number: 26174725.7
(22) Date of filing: 22.12.2022
(51) Int. Cl.: A61B 18/22

(54) **CATHETER SYSTEM INCLUDING ALIGNMENT ASSEMBLY FOR OPTICAL FIBER CONNECTORS IN MEDICAL LASER APPLICATIONS**

(30) Priority: 23.12.2021 US 202163293330 P; 21.12.2022 US 202218086408
(62) Divisional of application: 22854285.8
(71) Applicant: Boston Scientific Scimed, Inc., Maple Grove, Minnesota 55311 (US)
(72) Inventor: COOK, Christopher, A., Laguna Niguel, CA, 92677 (US); FANG, Itzhak, Irvine, CA, 92614 (US); ZALESKI, Richard, San Juan Capistrano, CA, 92675 (US); WANG, Ting Ting, Irvine, CA, 92620 (US); SWIFT, James, Foothill Ranch, CA, 92610 (US)
(74) Representative: Vossius & Partner Patentanwälte Rechtsanwälte mbB

(57) **Abstract**

The present invention discloses a method for aligning a light source within a catheter system for treating a vascular lesion within or adjacent to a vessel wall within a body of a patient, the catheter system including the single light source that generates light energy, the method comprising the steps of: initiating control of a plurality of hardware of the catheter system, the plurality of hardware including at least one of (i) a light guide that is configured to selectively receive light energy from the light source, (ii) a camera that captures an alignment of the light source within the light guide, (iii) a mover that is configured to selectively adjust a positioning of the light source within the light guide; performing a coarse alignment of the positioning of the light source within the light guide; checking at least one of (i) the alignment of the light source within the light guide, and (ii) a light energy level of the light source; and performing a fine alignment of the positioning of the light source within the light guide.

## Description

### RELATED APPLICATIONS

This Application is related to and claims priority on U.S. Provisional Patent Application Serial No. 63/293,330 filed on December 23, 2021, and entitled "HIGHLY ACCURATE ALIGNMENT MECHANISM, SYSTEM, ASSEMBLY, AND METHOD FOR OPTICAL FIBER CONNECTORS IN MEDICAL LASER APPLICATIONS", and on U.S. Patent Application Serial No. 18/086,408, filed on December 21, 2022, and entitled "CATHETER SYSTEM INCLUDING ALIGNMENT ASSEMBLY FOR OPTICAL FIBER CONNECTORS IN MEDICAL LASER APPLICATIONS". To the extent permissible, the contents of U.S. Application Serial No. 63/293,330 and U.S. Patent Application Serial No. 18/086,408 are incorporated in their entirety herein by reference.

### BACKGROUND

Vascular lesions within vessels in the body can be associated with an increased risk for major adverse events, such as myocardial infarction, embolism, deep vein thrombosis, stroke, and the like. Severe vascular lesions, such as severely calcified vascular lesions, can be challenging to treat and achieve patency for a physician in a clinical setting.

Vascular lesions may be treated using interventions such as drug therapy, balloon angioplasty, atherectomy, stent placement, and vascular graft bypass, to name a few. Such interventions may not always be ideal or may require subsequent treatment to address the lesion.

In modern medicine, lasers are increasingly utilized to treat a variety of pathologies as interest in less invasive treatment modalities intensifies. The physics behind lasers allows the same basic principles to be applied to many tissue types using slight modifications of the system. Laser energy can be safely and effectively used for lithotripsy, intravascular lithotripsy, for the treatment of various types of cancer, for many cosmetic and reconstructive procedures, and the ablation of abnormal conductive pathways. As some applications require access to constricted spaces, devices with tiny diameter fibers may be of interest. When the energy required for an application is high, device designers may have to find ways to direct the laser energy into a small face of the fiber while trying to stay under the damage threshold of the fiber optic material (damage threshold is the highest power/energy density per area, that the optic can withstand without causing damage).

Fiber optics cores have a much higher damage threshold than the jacket and alignment ferrule surrounding the fiber, and therefore if the laser beam energy falls outside the core, even into the cladding, the fiber will be damaged even if the power level is such that it can be conducted without damage through the fiber core.

Since the therapeutic application defines the energy level needs, the other parameter that designers can control to reduce the density is to increase the incidence area of the laser beam on the fiber face. While that reduces the power/energy density, it reduces the margins between the laser beam incidence area and the fiber core size.

The term commonly used to ensure that the beam and the fiber are concentric is the alignment of the beam to the fiber. When the margin above is small, the alignment must be very accurate to the level of single microns.

Devices encapsulating more than a single fiber (e.g., a fiber array) and a single laser beam that is being diverted to couple to them by a mechanism such as an acousto-optic modulator (AOM) or other multiplexing must fine-tune the alignment of the diverted beam in a way that would apply to all the fibers in the fiber array. In situations in which multiple beams are being directed towards a multiplicity of fibers simultaneously, the alignment does not only require beam location with fiber location alignment, but the line between the beams and the line between the target fibers must overlap each other.

Previous alignment methods utilized laser beams routed through an acousto-optic deflector (AOD) and correction of that alignment by adjusting a screw or piezo electric component that changes the direction of the laser source (or a mirror reflecting it on the path to the AOD), and therefore also affects the line on the fiber array plane, on which the AOD directs the beam. The problems with using AODs for beam deflection include energy loss due to the AOD and low coupling tolerance on the light guide. The incidence angle of the deflected beam (in higher angles of deflection) leads to an oval beam shape, which leads to less efficient optical coupling.

Medical devices introduce a specific challenge related to keeping the alignment accurate. Because of health hazards related to cross-contamination between patients, in many medical devices, there is a preference to design the device such that the part that is in contact with the patient (applied part) is a single-use device. When the fiber is encapsulated in the applied part, and the high-energy laser is not part of the single-use device, the alignment above must be maintained or re-established every time a new applied part device is connected to the laser source.

### SUMMARY

The present invention is directed toward a catheter system for placement within a blood vessel having a vessel wall. The catheter system can be used for treating a vascular lesion within or adjacent to the vessel wall within a body of a patient. The catheter system includes a light source, a receptacle assembly, a first light guide, a second light guide, a multiplexer, and an alignment assembly. The light source generates a source beam of light energy. The first light guide and the second light guide are coupled to the receptacle assembly, each light guide having a guide proximal end. The multiplexer receives the source beam from the light source, the multiplexer directing individual guide beams from the source beam to each of the guide proximal end of the first light guide and the guide proximal end of the second light guide. The alignment assembly adjusts the position of the receptacle assembly relative to the individual guide beams.

In certain embodiments, each of the guide proximal end of the first light guide and the guide proximal end of the second light guide have two rotational degrees of freedom.

In some embodiments, the receptacle assembly has three degrees of freedom.

In various embodiments, the receptacle assembly has three rotational degrees of freedom.

In certain embodiments, the multiplexer has at least one degree of freedom.

In some embodiments, the alignment assembly adjusts the position of the receptacle assembly relative to the individual guide beams in micrometer level adjustments.

In various embodiments, the alignment assembly adjusts the position of the receptacle assembly relative to the multiplexer.

In certain embodiments, the receptacle assembly is coupled to the alignment assembly.

In some embodiments, the multiplexer is coupled to the alignment assembly.

In various embodiments, the alignment assembly includes a camera that captures images of the guide proximal ends of each light guide so that the position of the individual guide beams relative to the guide proximal ends can be adjusted.

The present invention is also directed toward a catheter system for placement within a blood vessel having a vessel wall. The catheter system can be used for treating a vascular lesion within or adjacent to the vessel wall within a body of a patient. The catheter system includes a light source, a receptacle assembly, a first light guide, and a second light guide, a multiplexer, and an alignment assembly. The light source generates a source beam of light energy. The first light guide and the second light guide are coupled to the receptacle assembly, each light guide having a guide proximal end. The multiplexer receives the source beam from the light source, the multiplexer directing individual guide beams from the source beam to each of the guide proximal end of the first light guide and the guide proximal end of the second light guide. The alignment assembly adjusts the position of the receptacle assembly relative to the individual guide beams.

In some embodiments, the alignment assembly adjusts the position of the receptacle assembly relative to the individual guide beams in micrometer level corrections.

In various embodiments, the alignment assembly adjusts the position of the receptacle assembly relative to the multiplexer.

In certain embodiments, the receptacle assembly is coupled to the alignment assembly.

In some embodiments, each of the guide proximal ends is coupled to the alignment assembly so that the guide proximal ends have two rotational degrees of freedom.

In various embodiments, the receptacle assembly is coupled to the alignment assembly so that the receptacle assembly has three degrees of freedom.

In certain embodiments, the receptacle assembly is coupled to the alignment assembly so that the receptacle assembly has three rotational degrees of freedom.

In some embodiments, the multiplexer is coupled to the alignment assembly so that the multiplexer has at least one degree of freedom.

In various embodiments, the alignment assembly includes a camera that captures images of the guide proximal ends of each light guide so that the position of the individual guide beams relative to the guide proximal ends can be adjusted.

In certain embodiments, the catheter system further comprises a system controller including a processor that is configured to control operation of the light source to generate a single source beam in the form of pulses of light energy that are directed to the multiplexer; the system controller being further configured to control operation of the multiplexer so that a first guide beam is directed to the first light guide and a second guide beam is directed to the second light guide.

In some embodiments, the light source includes a laser.

In various embodiments, the catheter system further comprises a catheter shaft and a balloon that is coupled to the catheter shaft, the balloon including a balloon wall that defines a balloon interior, the balloon being configured to retain a balloon fluid within the balloon interior; wherein the first light guide and the second light guide are positioned at least partially within the balloon interior.

In certain embodiments, the balloon is selectively inflatable with the balloon fluid to expand to an inflated state, wherein when the balloon is in the inflated state the balloon wall is configured to be positioned substantially adjacent to the vascular lesion.

In some embodiments, the first light guide and the second light guide receive the light energy from the light source and guide the light energy from the light source into the balloon interior to generate plasma in the balloon fluid within the balloon interior, the plasma generation causing rapid bubble formation and imparting pressure waves upon the balloon wall adjacent to the vascular lesion.

In various embodiments, the multiplexer includes an optical element that splits the source beam into a first guide beam and a second guide beam.

In certain embodiments, the multiplexer further includes coupling optics that are configured to focus the first guide beam onto the first light guide and the second guide beam onto the second light guide.

In some embodiments, the first guide beam and the second guide beam are incident on the coupling optics with an angle between them.

In various embodiments, the catheter system further comprises a guide bundle that brings each of the first light guide and the second light guide closer together so that the first light guide and the second light guide are in a more compact form.

In certain embodiments, the receptacle assembly includes a receptacle assembly housing, a receptacle ferrule receiver, and a receptacle ferrule retainer.

In some embodiments, the receptacle ferrule retainer selectively locks each of the first light guide and the second light guide in desired locations within the receptacle ferrule receiver.

In various embodiments, the receptacle ferrule retainer is a plunger ball spring bridge including a plurality of plunger ball springs that are each configured to selectively lock each of the first light guide and the second light guide in desired locations within the receptacle ferrule receiver.

In certain embodiments, the alignment assembly includes a first stage selectively fixed to the receptacle assembly, the first stage being configured to rotate about one of a first axis and a second axis.

In some embodiments, the alignment assembly further includes a first stage knob that selectively engages a first rotary cam.

In various embodiments, the first rotary cam is coupled to a first mover.

In certain embodiments, the first mover drives the first rotary cam so that the first stage moves in a rotary motion.

In some embodiments, the alignment assembly includes a second stage selectively fixed to the first stage, the second stage being configured to lift and lower the first stage on a first axis.

In various embodiments, the alignment assembly includes a second stage knob that engages a second rotary cam.

In certain embodiments, the second rotary cam is coupled to a second mover.

In some embodiments, the second mover drives the second rotary cam so that the second stage lifts and lowers the first stage on a first axis.

The present invention is also directed toward a method for aligning a light source within a catheter system for treating a vascular lesion within or adjacent to a vessel wall within a body of a patient, the catheter system including a single light source that generates light energy. In various embodiments, the method can comprise the steps of initiating control of a plurality of hardware of the catheter system, the plurality of hardware including at least one of (i) a light guide that is configured to selectively receive light energy from the light source, (ii) a camera that captures an alignment of the light source within the light guide, (iii) a mover that is configured to selectively adjust a positioning of the light source within the light guide; performing a coarse alignment of the positioning of the light source within the light guide; checking at least one of (i) the alignment of the light source within the light guide, and (ii) a light energy level of the light source; and performing a fine alignment of the positioning of the light source within the light guide.

In various embodiments, the method further comprises the step of checking a light connector alignment.

In certain embodiments, the method further comprises the step of continuously monitoring the alignment of the light source within the light guide with the camera.

In some embodiments, the step of performing the coarse alignment includes loading hardcoded alignment settings of an alignment algorithm that is configured to autonomously align the light source with the light guide.

In various embodiments, the step of performing the coarse alignment includes loading a plurality of light guide reference locations and coordinates from the alignment algorithm.

In certain embodiments, the step of performing the coarse alignment includes initializing at least one of (i) the camera, (ii) an illumination source that is configured to illuminate an end face of the light guide, and (iii) a stage that is coupled to the light guide, the stage being configured to adjust the positioning of the light guide relative to the light source.

In some embodiments, the step of performing the coarse alignment includes moving the stage to a second stage location.

In various embodiments, the step of performing the coarse alignment includes capturing a plurality of captured images with the camera while the stage is in the second stage location.

In certain embodiments, the step of performing the coarse alignment includes determining if a plurality of reference targets are included in the plurality of captured images.

In some embodiments, if the plurality of reference targets are included in the plurality of captured images, the step of performing the coarse alignment includes detecting a plurality of light guide reference locations and coordinates with an alignment algorithm that is configured to autonomously align the light source with the light guide.

In various embodiments, if the plurality of reference targets is not included in the plurality of captured images, the step of performing the coarse alignment includes moving the stage to a second stage location and capturing a plurality of second captured images with the camera while the stage is in the second stage location.

In certain embodiments, the step of performing the coarse alignment includes checking the catheter system for system errors.

In some embodiments, if the catheter system has one or more system errors, the catheter system is updated, and error handling is initiated.

In various embodiments, if the catheter system does not have has one or more system errors, the step of performing the coarse alignment includes calculating a first tolerance region for a plurality of light guide reference locations and coordinates.

In certain embodiments, the step of performing the coarse alignment includes verifying that the alignment of the light source within the light guide is within the first tolerance region for the plurality of light guide reference locations and coordinates.

In some embodiments, if the alignment of the light source within the light guide is within the first tolerance region for the plurality of light guide reference locations and coordinates, the step of performing the coarse alignment is completed.

In various embodiments, if the alignment of the light source within the light guide is not within the first tolerance region for the plurality of light guide reference locations and coordinates, the step of performing the coarse alignment includes validating a plurality of alignment data.

In certain embodiments, if the validating of the plurality of alignment data results in a validation error, the catheter system is updated, and error handling is initiated.

In some embodiments, if the validating of the plurality of alignment data does not result in a validation error, the step of performing the coarse alignment further includes removing a plurality of camera offsets; and performing a linear regression with respect to a plurality of hardware offsets.

In various embodiments, the plurality of hardware offsets include at least one of a tilt offset, a y-axis offset, and an x-axis offset.

In certain embodiments, the step of performing the coarse alignment further includes positioning the plurality of hardware based on the plurality of hardware offsets.

In some embodiments, the step of performing the coarse alignment further includes moving the stage to a third stage location and capturing a plurality of third captured images with the camera while the stage is in the third stage location.

The present invention is further directed toward a catheter system for treating a vascular lesion within or adjacent to a vessel wall within a body of a patient. In certain embodiments, the catheter system comprises a light source that generates a source beam of light energy; a receptacle assembly; a first light guide, and a second light guide that is coupled to the receptacle assembly, each light guide having a guide proximal end; a multiplexer that receives the source beam from the light source, the multiplexer directing individual guide beams from the source beam to each of the guide proximal end of the first light guide and the guide proximal end of the second light guide; and an alignment assembly that adjusts the position of the receptacle assembly relative to the individual guide beams, the alignment assembly including a first mover that moves the receptacle assembly.

In various embodiments, the first mover includes at least one of a motor and an actuator.

In certain embodiments, the first mover includes a rack and pinion motor.

In some embodiments, the alignment assembly further includes a plurality of rollers coupled to the first mover, the plurality of rollers being configured to roll the first stage about the first axis.

In various embodiments, the alignment assembly includes a mechanical flexure that cooperates with the first mover to create angular displacement.

In certain embodiments, the alignment assembly includes a mechanical flexure that cooperates with the first mover to create linear displacement.

In some embodiments, the first mover includes a screw mechanism and a motor.

In various embodiments, the first mover includes one of a piezoelectric stack and an actuator.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a schematic cross-sectional view of an embodiment of a catheter system in accordance with various embodiments herein, the catheter system including a plurality of light guides, a multiplexer, and a guide bundle;
Figure 2 is a perspective partially exploded view of a portion of an embodiment of the catheter system, including an alignment assembly and a receptacle assembly;
Figure 3 is a front view of a portion of an embodiment of the catheter system, including another embodiment of the alignment assembly and the receptacle assembly;
Figure 4 is a perspective view of a portion of an embodiment of the catheter system, including one embodiment of the guide bundle;
Figure 5 is a perspective view of a portion of an embodiment of the catheter system, including one embodiment of the receptacle assembly;
Figure 6 is a perspective view of a portion of an embodiment of the catheter system, including yet another embodiment of the alignment assembly, the receptacle assembly, and the guide bundle;
Figure 7 is a rear view of a portion of an embodiment of the catheter system, including one embodiment of the alignment assembly and the receptacle assembly;
Figure 8 is a side view of a portion of an embodiment of the catheter system, including one embodiment of the alignment assembly and the receptacle assembly;
Figure 9 is a top view of a portion of an embodiment of the catheter system, including one embodiment of the alignment assembly and the receptacle assembly;
Figure 10 is a cross-sectional view of a portion of an embodiment of the catheter system taken on line 10-10 in Figure 6;
Figure 11 is a flow chart depicting one embodiment of a method for aligning a light source within a catheter system in accordance with various embodiments herein;
Figure 12 is a flow chart depicting one embodiment of a method for performing a coarse alignment of the light source within the catheter system;
Figure 13 is a flow chart depicting yet another embodiment of a method for aligning the light source within the catheter system;
Figure 14 is an illustration of one embodiment of an end face of the light guide captured by a camera during one embodiment of a method for aligning the light source within the catheter system;
Figure 15 is another illustration of the end face of the light guide captured by the camera during one embodiment of a method for aligning the light source within the catheter system;
Figure 16 is yet another illustration of the end face of the light guide captured by the camera during one embodiment of a method for aligning the light source within the catheter system;
Figure 17 is yet another illustration of the end face of the light guide captured by the camera during one embodiment of a method for aligning the light source within the catheter system; and
Figure 18 is an illustration of a user interface displaying an overlaid plane used during one embodiment of a method for aligning the light source within the catheter system.

While embodiments of the present invention are susceptible to various modifications and alternative forms, specifics thereof have been shown by way of examples and drawings, and are described in detail herein. It is understood, however, that the scope herein is not limited to the particular embodiments described. On the contrary, the intention is to cover modifications, equivalents, and alternatives falling within the spirit and scope herein.

### DESCRIPTION

Treatment of vascular lesions can reduce major adverse events or death in affected subjects. As referred to herein, a major adverse event is one that can occur anywhere within the body due to the presence of a vascular lesion. Major adverse events can include but are not limited to major adverse cardiac events, major adverse events in the peripheral or central vasculature, major adverse events in the brain, major adverse events in the musculature, or major adverse events in any of the internal organs.

For the treatment of vascular lesions, such as calcium deposits in arteries, it is generally beneficial to treat multiple closely spaced areas with a single insertion and positioning of a catheter balloon. To allow this to occur within an optical excitation system, such as within a laser-driven intravascular lithotripsy device, it is usually desirable to have a number of output channels, e.g., optical fibers and targets, for the treatment process, which can be distributed within the balloon. Since a high-power laser source is often the largest and most expensive component in the system, having a dedicated laser source for each optical fiber is unlikely to be feasible for a number of reasons, including packaging requirements, power consumption, thermal considerations, and economics. For such reasons, it can be advantageous to multiplex a single laser simultaneously and/or sequentially into a number of different optical fibers for treatment purposes. This allows the possibility for using all or a particular portion of the laser power from the single laser with each fiber.

Thus, the catheter systems and related methods are configured to provide a means to power multiple fiber optic channels in a laser-driven pressure wave-generating device that is designed to impart pressure onto and induce fractures in vascular lesions, such as calcified vascular lesions and/or fibrous vascular lesions, using a single light source. More particularly, the present invention includes a multiplexer that multiplexes a single light source, e.g., a single laser source, into one or more of multiple light guides, e.g., fiber optic channels, in a single-use device.

One of the problems of using optical fibers to transfer high-energy optical pulses is that there can be significant limitations on the amount of energy carried by the optical fiber due to physical damage concerns and nonlinear processes such as Stimulated Brillouin Scattering (SBS). For this reason, it may be advantageous to have the option of accessing multiple fibers, i.e., light guides, simultaneously in order to increase the amount of energy that can be delivered at one time without directing excessive energy through any single fiber. The present technology further allows a single, stable light source to be channeled sequentially through a plurality of light guides with a variable number.

In various embodiments, the catheter systems and related methods disclosed herein can include a catheter configured to advance to vascular lesions, such as calcified vascular lesions or a fibrous vascular lesions, located at a treatment site within or adjacent to a blood vessel within a body of a patient. The catheter includes a catheter shaft, and an inflatable balloon that is coupled and/or secured to the catheter shaft. The balloon can include a balloon wall that defines a balloon interior. The balloon can be configured to receive a balloon fluid within the balloon interior to expand from a deflated state suitable for advancing the catheter through a patient's vasculature, to an inflated state suitable for anchoring the catheter in position relative to the treatment site.

The catheter systems also include the plurality of light guides disposed along the catheter shaft and within the balloon interior of the balloon. Each light guide can be configured for generating pressure waves within the balloon to disrupt the vascular lesions. In particular, the catheter systems utilize light energy from the light source to create a localized plasma in the balloon fluid within the balloon interior of the balloon at or near a guide distal end of the light guide disposed in the balloon located at the treatment site. As such, the light guide can sometimes be referred to as, or can be said to incorporate a "plasma generator" at or near the guide distal end of the light guide that is positioned within the balloon interior of the balloon located at the treatment site. The creation of the localized plasma can initiate a pressure wave and can initiate the rapid formation of one or more high energy bubbles that can rapidly expand to a maximum size and then dissipate through a cavitation event that can launch a pressure wave upon collapse. The rapid expansion of the plasma-induced bubbles can generate one or more pressure waves within the balloon fluid retained within the balloon interior of the balloon and thereby impart pressure waves onto and induce fractures in the vascular lesions at the treatment site within or adjacent to the blood vessel wall within the body of the patient. It is appreciated that the guide distal end of each of the plurality of light guides can be positioned in any suitable location relative to the length of the balloon to more effectively and precisely impart pressure waves to disrupt the vascular lesions at the treatment site.

In some embodiments, the light source can be configured to provide sub-millisecond pulses of light energy to initiate the plasma formation in the balloon fluid within the balloon to cause rapid bubble formation and to impart pressure waves upon the balloon wall at the treatment site. Thus, the pressure waves can transfer mechanical energy through an incompressible balloon fluid to the treatment site to impart a fracture force on the vascular lesions. Without wishing to be bound by any particular theory, it is believed that the rapid change in balloon fluid momentum upon the balloon wall that is in contact with the intravascular lesion is transferred to the intravascular lesion to induce fractures to the lesion.

Importantly, as noted above, the catheter systems and related methods include the multiplexer that multiplexes a single light source into one or more of the light guides in a single-use device to enable the treatment of multiple closely spaced areas with a single insertion and positioning of a catheter balloon.

As used herein, the terms "intravascular lesion" and "vascular lesion" are used interchangeably unless otherwise noted. As such, the intravascular lesions and/or the vascular lesions are sometimes referred to herein simply as "lesions."

Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention, as illustrated in the accompanying drawings. The same or similar nomenclature and/or reference indicators will be used throughout the drawings, and the following detailed description to refer to the same or like parts.

In the interest of clarity, not all of the routine features of the implementations described herein are shown and described. It is appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is appreciated that such a development effort might be complex and time-consuming, but would nevertheless be a routine undertaking of engineering for those of ordinary skill in the art having the benefit of this disclosure.

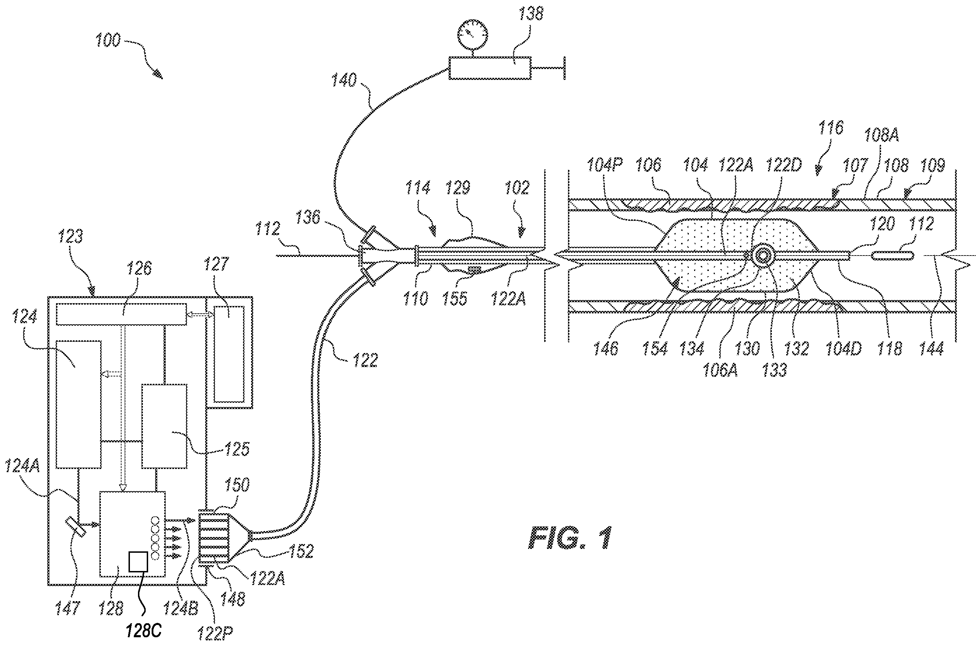

The catheter systems disclosed herein can include many different forms. Referring now to Figure 1, a schematic cross-sectional view is shown of a catheter system 100 in accordance with various embodiments. The catheter system 100 is suitable for imparting pressure waves to induce fractures in one or more vascular lesions within or adjacent to a vessel wall of a blood vessel within a body of a patient. In the embodiment illustrated in Figure 1, the catheter system 100 can include one or more of a catheter 102, a light guide bundle 122 including one or more (and preferably a plurality of) light guides 122A, a source manifold 136, a fluid pump 138, a system console 123 including one or more of a light source 124, a power source 125, a system controller 126, a graphic user interface 127 (a "GUI"), and a multiplexer 128, and a handle assembly 129. Alternatively, the catheter system 100 can include more components or fewer components than those specifically illustrated and described in relation to Figure 1.

The catheter 102 is configured to move to a treatment site 106 within or adjacent to a vessel wall 108A of a blood vessel 108 within a body 107 of a patient 109. The treatment site 106 can include one or more vascular lesions 106A, such as calcified vascular lesions, for example. Additionally, or in the alternative, the treatment site 106 can include vascular lesions 106A, such as fibrous vascular lesions.

The catheter 102 can include an inflatable balloon 104 (sometimes referred to herein simply as a "balloon"), a catheter shaft 110, and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter shaft 110 can also include a guidewire lumen 118, which is configured to move over the guidewire 112. As utilized herein, the guidewire lumen 118 defines a conduit through which the guidewire 112 extends. The catheter shaft 110 can further include an inflation lumen (not shown) and/or various other lumens for various other purposes. In some embodiments, the catheter 102 can have a distal end opening 120 and can accommodate and be tracked over the guidewire 112 as the catheter 102 is moved and positioned at or near the treatment site 106. In some embodiments, the balloon proximal end 104P can be coupled to the catheter shaft 110, and the balloon distal end 104D can be coupled to the guidewire lumen 118.

The balloon 104 includes a balloon wall 130 that defines a balloon interior 146. The balloon 104 can be selectively inflated with a balloon fluid 132 to expand from a deflated state suitable for advancing the catheter 102 through a patient's vasculature, to an inflated state (as shown in Figure 1) suitable for anchoring the catheter 102 in position relative to the treatment site 106. Stated in another manner, when the balloon 104 is in the inflated state, the balloon wall 130 of the balloon 104 is configured to be positioned substantially adjacent to the treatment site 106, i.e., to the vascular lesion(s) 106A at the treatment site 106. It is appreciated that although Figure 1 illustrates the balloon wall 130 of the balloon 104 being shown spaced apart from the treatment site 106 of the blood vessel 108 when in the inflated state, this is done merely for ease of illustration. It is recognized that the balloon wall 130 of the balloon 104 will typically be substantially directly adjacent to and/or abutting the treatment site 106 when the balloon 104 is in the inflated state.

The balloon 104 suitable for use in the catheter system 100 includes those that can be passed through the vasculature of a patient 109 when in the deflated state. In some embodiments, the balloon 104 is made from silicone. In other embodiments, the balloon 104 can be made from polydimethylsiloxane (PDMS), polyurethane, polymers such as PEBAX^{™} material, nylon, or any other suitable material.

The balloon 104 can have any suitable diameter (in the inflated state). In various embodiments, the balloon 104 can have a diameter (in the inflated state) ranging from less than one millimeter (mm) up to 25 mm. In some embodiments, the balloon 104 can have a diameter (in the inflated state) ranging from at least 1.5 mm up to 14 mm. In some embodiments, the balloon 104 can have a diameter (in the inflated state) ranging from at least two mm up to five mm.

In some embodiments, the balloon 104 can have a length ranging from at least three mm to 300 mm. More particularly, in some embodiments, the balloon 104 can have a length ranging from at least eight mm to 200 mm. It is appreciated that a balloon 104 having a relatively longer length can be positioned adjacent to larger treatment sites 106, and, thus, may be usable for imparting pressure waves onto and inducing fractures in larger vascular lesions 106A or multiple vascular lesions 106A at precise locations within the treatment site 106. It is further appreciated that a longer balloon 104 can also be positioned adjacent to multiple treatment sites 106 at any one given time.

The balloon 104 can be inflated to inflation pressures of between approximately one atmosphere (atm) and 70 atm. In some embodiments, the balloon 104 can be inflated to inflation pressures of from at least 20 atm to 60 atm. In other embodiments, the balloon 104 can be inflated to inflation pressures of from at least six atm to 20 atm. In still other embodiments, the balloon 104 can be inflated to inflation pressures of from at least three atm to 20 atm. In yet other embodiments, the balloon 104 can be inflated to inflation pressures of from at least two atm to ten atm.

The balloon 104 can have various shapes, including, but not to be limited to, a conical shape, a square shape, a rectangular shape, a spherical shape, a conical/square shape, a conical/spherical shape, an extended spherical shape, an oval shape, a tapered shape, a bone shape, a stepped diameter shape, an offset shape, or a conical offset shape. In some embodiments, the balloon 104 can include a drug-eluting coating or a drug-eluting stent structure. The drug-eluting coating or drug-eluting stent can include one or more therapeutic agents including anti-inflammatory agents, anti-neoplastic agents, anti-angiogenic agents, and the like.

The balloon fluid 132 can be a liquid or a gas. Some examples of the balloon fluid 132 suitable for use can include, but are not limited to one or more of water, saline, contrast medium, fluorocarbons, perfluorocarbons, gases, such as carbon dioxide, or any other suitable balloon fluid 132. In some embodiments, the balloon fluid 132 can be used as a base inflation fluid. In some embodiments, the balloon fluid 132 can include a mixture of saline to contrast medium in a volume ratio of approximately 50:50. In other embodiments, the balloon fluid 132 can include a mixture of saline to contrast medium in a volume ratio of approximately 25:75. In still other embodiments, the balloon fluid 132 can include a mixture of saline to contrast medium in a volume ratio of approximately 75:25. However, it is understood that any suitable ratio of saline to contrast medium can be used. The balloon fluid 132 can be tailored on the basis of composition, viscosity, and the like so that the rate of travel of the pressure waves are appropriately manipulated. In certain embodiments, the balloon fluid 132 suitable for use herein is biocompatible. A volume of balloon fluid 132 can be tailored by the chosen light source 124 and the type of balloon fluid 132 used.

In some embodiments, the contrast agents used in the contrast media can include, but are not to be limited to, iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine-based contrast agents can be used. Suitable non-iodine-containing contrast agents can include gadolinium (III)-based contrast agents. Suitable fluorocarbon and perfluorocarbon agents can include, but are not to be limited to, agents such as perfluorocarbon dodecafluoropentane (DDFP, C5F12).

The balloon fluids 132 can include those that include absorptive agents that can selectively absorb light in the ultraviolet region (e.g., at least ten nanometers (nm) to 400 nm), the visible region (e.g., at least 400 nm to 780 nm), or the near-infrared region (e.g., at least 780 nm to 2.5 µm) of the electromagnetic spectrum. Suitable absorptive agents can include those with absorption maxima along the spectrum from at least ten nm to 2.5 µm. Alternatively, the balloon fluid 132 can include absorptive agents that can selectively absorb light in the mid-infrared region (e.g., at least 2.5 µm to 15 µm), or the far-infrared region (e.g., at least 15 µm to one mm) of the electromagnetic spectrum. In various embodiments, the absorptive agent can be those that have an absorption maximum matched with the emission maximum of the laser used in the catheter system 100. By way of non-limiting examples, various lasers described herein can include neodymium:yttrium-aluminum-garnet (Nd:YAG - emission maximum = 1064 nm) lasers, holmium:YAG (Ho:YAG - emission maximum = 2.1 µm) lasers, or erbium:YAG (Er:YAG - emission maximum = 2.94 µm) lasers. In some embodiments, the absorptive agents can be water-soluble. In other embodiments, the absorptive agents are not water-soluble. In some embodiments, the absorptive agents used in the balloon fluids 132 can be tailored to match the peak emission of the light source 124. Various light sources 124 having emission wavelengths of at least ten nanometers to one millimeter are discussed elsewhere herein.

The catheter shaft 110 of the catheter 102 can be coupled to the one or more light guides 122A of the light guide bundle 122 that are in optical communication with the light source 124. The light guide(s) 122A can be disposed along the catheter shaft 110 and within the balloon 104. Each of the light guides 122A can have a guide distal end 122D that is at any suitable longitudinal position relative to a length of the balloon 104. In some embodiments, each light guide 122A can be an optical fiber, and the light source 124 can be a laser. The light source 124 can be in optical communication with the light guides 122A at the proximal portion 114 of the catheter system 100. More particularly, as described in detail herein, the light source 124 can selectively, simultaneously, sequentially, and/or alternatively be in optical communication with each of the light guides 122A in any desired combination, order and/or pattern due to the presence and operation of the multiplexer 128.

In some embodiments, the catheter shaft 110 can be coupled to multiple light guides 122A, such as a first light guide, a second light guide, a third light guide, etc., which can be disposed at any suitable position about the guidewire lumen 118 and/or the catheter shaft 110. For example, in certain non-exclusive embodiments, two light guides 122A can be spaced apart by approximately 180 degrees about the circumference of the guidewire lumen 118 and/or the catheter shaft 110; three light guides 122A can be spaced apart by approximately 120 degrees about the circumference of the guidewire lumen 118 and/or the catheter shaft 110; or four light guides 122A can be spaced apart by approximately 90 degrees about the circumference of the guidewire lumen 118 and/or the catheter shaft 110. Still alternatively, multiple light guides 122A need not be uniformly spaced apart from one another about the circumference of the guidewire lumen 118 and/or the catheter shaft 110. More particularly, the light guides 122A can be disposed either uniformly or non-uniformly about the guidewire lumen 118 and/or the catheter shaft 110 to achieve the desired effect in the desired locations.

The catheter system 100 and/or the light guide bundle 122 can include any number of light guides 122A in optical communication with the light source 124 at the proximal portion 114, and with the balloon fluid 132 within the balloon interior 146 of the balloon 104 at the distal portion 116. For example, in some embodiments, the catheter system 100 and/or the light guide bundle 122 can include from one light guide 122A to five light guides 122A. In other embodiments, the catheter system 100 and/or the light guide bundle 122 can include from five light guides 122A to fifteen light guides 122A. In yet other embodiments, the catheter system 100 and/or the light guide bundle 122 can include from ten light guides 122A to thirty light guides 122A. Alternatively, in still other embodiments, the catheter system 100 and/or the light guide bundle 122 can include greater than 30 light guides 122A.

The light guides 122A can have any suitable design for purposes of generating plasma and/or pressure waves in the balloon fluid 132 within the balloon interior 146. In certain embodiments, the light guides 122A can include an optical fiber or flexible light pipe. The light guides 122A can be thin and flexible and can allow light signals to be sent with very little loss of strength. The light guides 122A can include a core surrounded by a cladding about its circumference. In some embodiments, the core can be a cylindrical core or a partially cylindrical core. The core and cladding of the light guides 122A can be formed from one or more materials, including but not limited to one or more types of glass, silica, or one or more polymers. The light guides 122A may also include a protective coating, such as a polymer. It is appreciated that the index of refraction of the core will be greater than the index of refraction of the cladding.

Each light guide 122A can guide light energy along its length from a guide proximal end 122P to the guide distal end 122D, having at least one optical window (not shown) that is positioned within the balloon interior 146.

The light guides 122A can assume many configurations about and/or relative to the catheter shaft 110 of the catheter 102. In some embodiments, the light guides 122A can run parallel to the longitudinal axis 144 of the catheter shaft 110. In some embodiments, the light guides 122A can be physically coupled to the catheter shaft 110. In other embodiments, the light guides 122A can be disposed along a length of an outer diameter of the catheter shaft 110. In yet other embodiments, the light guides 122A can be disposed within one or more light guide lumens within the catheter shaft 110.

The light guides 122A can also be disposed at any suitable positions about the circumference of the guidewire lumen 118 and/or the catheter shaft 110, and the guide distal end 122D of each of the light guides 122A can be disposed at any suitable longitudinal position relative to the length of the balloon 104 and/or relative to the length of the guidewire lumen 118 to more effectively and precisely impart pressure waves for purposes of disrupting the vascular lesions 106A at the treatment site 106.

In certain embodiments, the light guides 122A can include one or more photoacoustic transducers 154, where each photoacoustic transducer 154 can be in optical communication with the light guide 122A within which it is disposed. In some embodiments, the photoacoustic transducers 154 can be in optical communication with the guide distal end 122D of the light guide 122A. Additionally, in such embodiments, the photoacoustic transducers 154 can have a shape that corresponds with and/or conforms to the guide distal end 122D of the light guide 122A.

The photoacoustic transducer 154 is configured to convert light energy into an acoustic wave at or near the guide distal end 122D of the light guide 122A. The direction of the acoustic wave can be tailored by changing an angle of the guide distal end 122D of the light guide 122A.

In certain embodiments, the photoacoustic transducers 154 disposed at the guide distal end 122D of the light guide 122A can assume the same shape as the guide distal end 122D of the light guide 122A. For example, in certain non-exclusive embodiments, the photoacoustic transducer 154 and/or the guide distal end 122D can have a conical shape, a convex shape, a concave shape, a bulbous shape, a square shape, a stepped shape, a half-circle shape, an ovoid shape, and the like. The light guide 122A can further include additional photoacoustic transducers 154 disposed along one or more side surfaces of the length of the light guide 122A.

In some embodiments, the light guides 122A can further include one or more diverting features or "diverters" (not shown in Figure 1) within the light guide 122A that are configured to direct light to exit the light guide 122A toward a side surface which can be located at or near the guide distal end 122D of the light guide 122A, and toward the balloon wall 130. A diverting feature can include any feature of the system that diverts light energy from the light guide 122A away from its axial path toward a side surface of the light guide 122A. Additionally, the light guides 122A can each include one or more light windows disposed along the longitudinal or circumferential surfaces of each light guide 122A and in optical communication with a diverting feature. Stated in another manner, the diverting features can be configured to direct light energy in the light guide 122A toward a side surface that is at or near the guide distal end 122D, where the side surface is in optical communication with a light window. The light windows can include a portion of the light guide 122A that allows light energy to exit the light guide 122A from within the light guide 122A, such as a portion of the light guide 122A lacking a cladding material on or about the light guide 122A.

Examples of the diverting features suitable for use include a reflecting element, a refracting element, and a fiber diffuser. The diverting features suitable for focusing light energy away from the tip of the light guides 122A can include, but are not to be limited to, those having a convex surface, a gradient-index (GRIN) lens, and a mirror focus lens. Upon contact with the diverting feature, the light energy is diverted within the light guide 122A to one or more of a plasma generator 133 and the photoacoustic transducer 154 that is in optical communication with a side surface of the light guide 122A. As noted, the photoacoustic transducer 154 then converts light energy into an acoustic wave that extends away from the side surface of the light guide 122A.

The source manifold 136 can be positioned at or near the proximal portion 114 of the catheter system 100. The source manifold 136 can include one or more proximal end openings that can receive the one or more light guides 122A of the light guide bundle 122, the guidewire 112, and/or an inflation conduit 140 that is coupled in fluid communication with the fluid pump 138. The catheter system 100 can also include the fluid pump 138 that is configured to inflate the balloon 104 with the balloon fluid 132, i.e., via the inflation conduit 140, as needed.

As noted above, in the embodiment illustrated in Figure 1, the system console 123 includes one or more of the light source 124, the power source 125, the system controller 126, the GUI 127, and the multiplexer 128. Alternatively, the system console 123 can include more components or fewer components than those specifically illustrated in Figure 1. For example, in certain non-exclusive alternative embodiments, the system console 123 can be designed without the GUI 127. Still alternatively, one or more of the light source 124, the power source 125, the system controller 126, the GUI 127, and the multiplexer 128 can be provided within the catheter system 100 without the specific need for the system console 123.

As shown, the system console 123, and the components included therewith, is operatively coupled to the catheter 102, the light guide bundle 122, and the remainder of the catheter system 100. For example, in some embodiments, as illustrated in Figure 1, the system console 123 can include a console connection aperture 148 (also sometimes referred to generally as a "socket") by which the light guide bundle 122 is mechanically coupled to the system console 123. In such embodiments, the light guide bundle 122 can include a guide coupling housing 150 (also sometimes referred to generally as a "ferrule") that houses a portion, e.g., the guide proximal end 122P, of each of the light guides 122A. The guide coupling housing 150 is configured to fit and be selectively retained within the console connection aperture 148 to provide the mechanical coupling between the light guide bundle 122 and the system console 123.

The light guide bundle 122 can also include a guide bundle 152 (or "shell") that brings each of the individual light guides 122A closer together so that the light guides 122A and/or the light guide bundle 122 can be in a more compact form as it extends with the catheter 102 into the blood vessel 108 during use of the catheter system 100.

The light source 124 can be selectively and/or alternatively coupled in optical communication with each of the light guides 122A, i.e., to the guide proximal end 122P of each of the light guides 122A, in the light guide bundle 122. In particular, the light source 124 is configured to generate light energy in the form of a source beam 124A, such as a pulsed source beam, that can be selectively and/or alternatively directed to and received by each of the light guides 122A in the light guide bundle 122 in any desired combination, order, sequence and/or pattern. More specifically, as described in greater detail herein below, the source beam 124A from the light source 124 is directed through the multiplexer 128 such that individual guide beams 124B (or "multiplexed beams") can be selectively and/or alternatively directed into and received by each of the light guides 122A in the light guide bundle 122. In particular, each pulse of the light source 124, i.e., each pulse of the source beam 124A, can be directed through the multiplexer 128 to generate one or more separate guide beams 124B (only one is shown in Figure 1) that are selectively and/or alternatively directed to one or more of the light guides 122A in the light guide bundle 122.

The light source 124 can have any suitable design. In certain embodiments, the light source 124 can be configured to provide sub-millisecond pulses of light energy from the light source 124 that are focused onto a small spot in order to couple it into the guide proximal end 122P of the light guide 122A. Such pulses of light energy are then directed and/or guided along the light guides 122A to a location within the balloon interior 146 of the balloon 104, thereby inducing plasma formation in the balloon fluid 132 within the balloon interior 146 of the balloon 104, e.g., via the plasma generator 133 that can be located at the guide distal end 122D of the light guide 122A. In particular, the light emitted at the guide distal end 122D of the light guide 122A energizes the plasma generator 133 to form the plasma within the balloon fluid 132 within the balloon interior 146. The plasma formation causes rapid bubble formation, and imparts pressure waves upon the treatment site 106. An exemplary plasma-induced bubble 134 is illustrated in Figure 1.

In various non-exclusive alternative embodiments, the sub-millisecond pulses of light energy from the light source 124 can be delivered to the treatment site 106 at a frequency of between approximately one hertz (Hz) and 5000 Hz, between approximately 30 Hz and 1000 Hz, between approximately ten Hz and 100 Hz, or between approximately one Hz and 30 Hz. Alternatively, the sub-millisecond pulses of light energy can be delivered to the treatment site 106 at a frequency that can be greater than 5000 Hz or less than one Hz, or any other suitable range of frequencies.

It is appreciated that although the light source 124 is typically utilized to provide pulses of light energy, the light source 124 can still be described as providing a single source beam 124A, i.e., a single pulsed source beam.

The light sources 124 suitable for use herein can include various types of light sources, including lasers and lamps. Suitable lasers can include short pulse lasers on the sub-millisecond timescale. In some embodiments, the light source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths, and energy levels that can be employed to achieve plasma in the balloon fluid 132 of the catheter 102. In various non-exclusive alternative embodiments, the pulse widths can include those falling within a range including from at least ten ns to 3000 ns, at least 20 ns to 100 ns, or at least one ns to 500 ns. Alternatively, any other suitable pulse width range can be used.

Exemplary nanosecond lasers can include those within the UV to IR spectrum, spanning wavelengths of about ten nanometers (nm) to one millimeter (mm). In some embodiments, the light sources 124 suitable for use in the catheter system 100 can include those capable of producing light at wavelengths of from at least 750 nm to 2000 nm. In other embodiments, the light sources 124 can include those capable of producing light at wavelengths of from at least 700 nm to 3000 nm. In still other embodiments, the light sources 124 can include those capable of producing light at wavelengths of from at least 100 nm to ten micrometers (µm). Nanosecond lasers can include those having repetition rates of up to 200 kHz. In some embodiments, the laser can include a Q-switched thulium:yttrium-aluminum-garnet (Tm:YAG) laser. In other embodiments, the laser can include a neodymium:yttrium-aluminum-garnet (Nd:YAG) laser, holmium:yttrium-aluminum-garnet (Ho:YAG) laser, erbium:yttrium-aluminum-garnet (Er:YAG) laser, excimer laser, helium-neon laser, carbon dioxide laser, as well as doped, pulsed, fiber lasers.

The catheter system 100 can generate pressure waves having maximum pressures in the range of at least one megapascal (MPa) to 100 MPa. The maximum pressure generated by a particular catheter system 100 will depend on the light source 124, the absorbing material, the bubble expansion, the propagation medium, the balloon material, and other factors. In various non-exclusive alternative embodiments, the catheter system 100 can generate pressure waves having maximum pressures in the range of at least approximately two MPa to 50 MPa, at least approximately two MPa to 30 MPa, or at least approximately 15 MPa to 25 MPa.

The pressure waves can be imparted upon the treatment site 106 from a distance within a range from at least approximately 0.1 millimeters (mm) to greater than approximately 25 mm extending radially from the energy guides 122A when the catheter 102 is placed at the treatment site 106. In various non-exclusive alternative embodiments, the pressure waves can be imparted upon the treatment site 106 from a distance within a range from at least approximately ten mm to 20 mm, at least approximately one mm to ten mm, at least approximately 1.5 mm to four mm, or at least approximately 0.1 mm to ten mm extending radially from the energy guides 122A when the catheter 102 is placed at the treatment site 106. In other embodiments, the pressure waves can be imparted upon the treatment site 106 from another suitable distance that is different than the foregoing ranges. In some embodiments, the pressure waves can be imparted upon the treatment site 106 within a range of at least approximately two MPa to 30 MPa at a distance from at least approximately 0.1 mm to ten mm. In some embodiments, the pressure waves can be imparted upon the treatment site 106 from a range of at least approximately two MPa to 25 MPa at a distance from at least approximately 0.1 mm to ten mm. Still alternatively, other suitable pressure ranges and distances can be used.

The power source 125 is electrically coupled to and is configured to provide necessary power to each of the light source 124, the system controller 126, the GUI 127, the multiplexer 128, and the handle assembly 129. The power source 125 can have any suitable design for such purposes.

The system controller 126 is electrically coupled to and receives power from the power source 125. Additionally, the system controller 126 is coupled to and is configured to control operation of each of the light source 124, the GUI 127, and the multiplexer 128. The system controller 126 can include one or more processors or circuits for purposes of controlling the operation of at least the light source 124, the GUI 127, and the multiplexer 128. For example, the system controller 126 can control the light source 124 for generating pulses of light energy as desired and/or at any desired firing rate. Subsequently, the system controller 126 can then control the multiplexer 128 so that the light energy from the light source 124, i.e., the source beam 124A, can be effectively and accurately multiplexed so as to be selectively and/or alternatively directed to each of the light guides 122A in the form of individual guide beams 124B in a desired manner.

The system controller 126 can further be configured to control the operation of other components of the catheter system 100, such as the positioning of the catheter 102 adjacent to the treatment site 106, the inflation of the balloon 104 with the balloon fluid 132, etc. Further, or in the alternative, the catheter system 100 can include one or more additional controllers that can be positioned in any suitable manner to control the various operations of the catheter system 100. For example, in certain embodiments, an additional controller and/or a portion of the system controller 126 can be positioned and/or incorporated within the handle assembly 129.

The GUI 127 is accessible by the user or operator of the catheter system 100. Additionally, the GUI 127 is electrically connected to the system controller 126. With such a design, the GUI 127 can be used by the user or operator to ensure that the catheter system 100 is effectively utilized to impart pressure onto and induce fractures into the vascular lesions 106A at the treatment site 106. The GUI 127 can provide the user or operator with information that can be used before, during, and after the use of the catheter system 100. In one embodiment, the GUI 127 can provide static visual data and/or information to the user or operator. In addition, or in the alternative, the GUI 127 can provide dynamic visual data and/or information to the user or operator, such as video data or any other data that changes over time during the use of the catheter system 100. In various embodiments, the GUI 127 can include one or more colors, different sizes, varying brightness, etc., that may act as alerts to the user or operator. Additionally, or in the alternative, the GUI 127 can provide audio data or information to the user or operator. The specifics of the GUI 127 can vary depending upon the design requirements of the catheter system 100, or the specific needs, specifications, and/or desires of the user or operator.

As provided herein, the multiplexer 128 is configured to selectively and/or alternatively direct light energy from the light source 124 to each of the light guides 122A in the light guide bundle 122. More particularly, the multiplexer 128 is configured to receive light energy from a single light source 124, such as a single source beam 124A from a single laser source, and selectively and/or alternatively direct such light energy in the form of individual guide beams 124B to each of the light guides 122A in the light guide bundle 122 in any desired combination (i.e., simultaneously direct light energy through multiple light guides 122A), sequence, order and/or pattern. As such, the multiplexer 128 enables a single light source 124 to be channeled simultaneously and/or sequentially through a plurality of light guides 122A such that the catheter system 100 is able to impart pressure onto and induce fractures in vascular lesions at the treatment site 106 within or adjacent to the vessel wall 108A of the blood vessel 108 in a desired manner. Additionally, as shown, the catheter system 100 can include one or more optical elements 147 for purposes of directing the light energy in the form of the source beam 124A from the light source 124 to the multiplexer 128. The multiplexer 128 can have at least a single degree of freedom with respect to the axes shown and described herein.

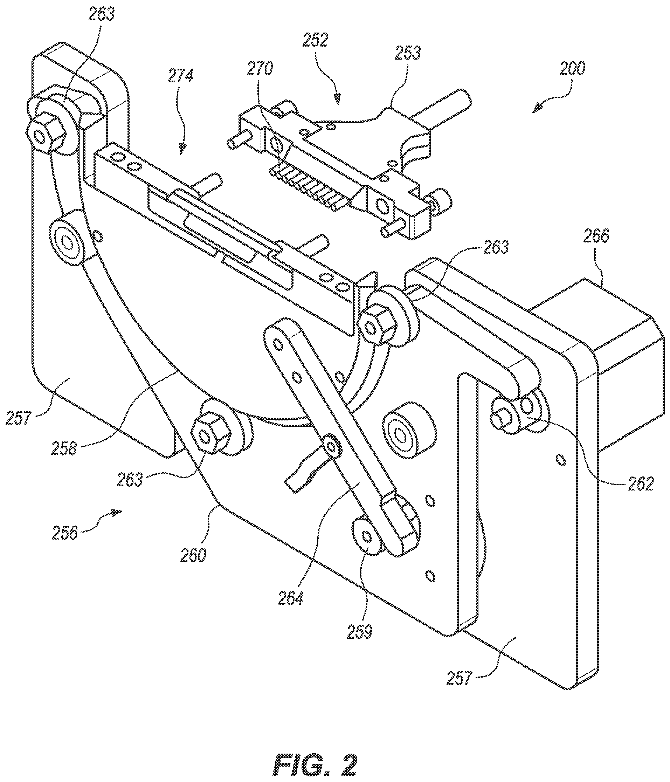

The multiplexer 128 can have any suitable design for purposes of selectively and/or alternatively directing the light energy from the light source 124 to each of the light guides 122A of the light guide bundle 122. In some embodiments, the multiplexer 128 can include a camera 128C. In other embodiments, the camera 128C can be positioned outside the multiplexer 128. In certain embodiments, the camera 128C can be included as part of an alignment assembly 256 (illustrated in Figure 2).

The camera 128C can vary depending on the design requirements of the catheter system 100. For example, in some embodiments, the camera 128C can include an illuminating source such as a light-emitting diode (or the illuminating source can be separate from the camera 128C). The camera 128C can capture images of the guide proximal end 122P of the light guide 122A so that the alignment of the individual guide beams 124B relative to the guide proximal end 122P can be adjusted. The system controller 126 can generate an X-Y-Z reference plane of coordinates using the images captured by the camera 128C. The camera 128C can be utilized machine vision for detecting a first picture, once the alignment hardware is moved to a first position. One or more cameras 128C can capture images taken at the first position. The camera 128C can continue to capture images until all the reference targets are captured. The system controller 126 can generate camera offsets based on the images generated by the camera 128C.

As shown in Figure 1, the handle assembly 129 can be positioned at or near the proximal portion 114 of the catheter system 100, and/or near the source manifold 136. In this embodiment, the handle assembly 129 is coupled to the balloon 104 and is positioned spaced apart from the balloon 104. Alternatively, the handle assembly 129 can be positioned at another suitable location.

The handle assembly 129 is handled and used by the user or operator to operate, position, and control the catheter 102. The design and specific features of the handle assembly 129 can vary to suit the design requirements of the catheter system 100. In the embodiment illustrated in Figure 1, the handle assembly 129 is separate from, but in electrical and/or fluid communication with one or more of the system controller 126, the light source 124, the fluid pump 138, the GUI 127, and the multiplexer 128. In some embodiments, the handle assembly 129 can integrate and/or include at least a portion of the system controller 126 within an interior of the handle assembly 129. For example, as shown, in certain such embodiments, the handle assembly 129 can include circuitry 155 that can form at least a portion of the system controller 126. In one embodiment, the circuitry 155 can include a printed circuit board having one or more integrated circuits, or any other suitable circuitry. In an alternative embodiment, the circuitry 155 can be omitted, or can be included within the system controller 126, which in various embodiments can be positioned outside of the handle assembly 129, e.g., within the system console 123. It is understood that the handle assembly 129 can include fewer or additional components than those specifically illustrated and described herein.

Figure 2 is a perspective partially exploded view of a portion of an embodiment of the catheter system 200, including the guide bundle 252, an alignment assembly 256, and a receptacle assembly 274. As shown in the embodiment displayed in Figure 2, the guide bundle 252 is detached from the alignment assembly 256 and the receptacle assembly 274.

The design of the catheter system 200 can be substantially similar to the embodiments illustrated and described herein. It is appreciated that various components of the catheter system 200, such as shown in Figure 1, are not illustrated in Figure 2 for clarity and ease of illustration, such as the multiplexer 128 (illustrated in Figure 1). However, it is appreciated that the catheter system 200 will likely include some, if not all, of such components.

As shown in Figure 2, the catheter system 200 again includes the guide bundle 252, including a guide bundle housing 253 that brings each of the individual light guides 122A (illustrated in Figure 1) closer together so that the light guides 122A and/or the guide bundle 252 can be in a more compact form as it extends with the catheter 102 (illustrated in Figure 1) into the blood vessel 108 (illustrated in Figure 1) during use of the catheter system 200. The guide bundle 252 can also include guide bundle ferrules 270 configured to optically couple the guide beams 124B (illustrated in Figure 1) from the multiplexer 128 into the corresponding light guides 122A.

The alignment assembly 256 can be configured to accurately adjust the alignment and/or positioning between the guide beams 124B that exit the multiplexer 128 and each of the corresponding individual light guides 122A that receive the matching guide beams 124B. The alignment assembly 256 can adjust the alignment and/or positioning down to micrometer level steps of adjustment for any of the individual light guides 122A. In other words, the alignment assembly 256 can adjust the position of the individual guide beams 124B relative to the receptacle assembly 274 (or any other suitable component of the catheter system 200) by single-digit micrometer steps. The alignment assembly 256 can grossly adjust the alignment and/or positioning of various components of the catheter system 200 upon initial connection of the guide bundle 252.

The alignment assembly 256 can align the light energy from the light source 124 (illustrated in Figure 1) so that the light energy in the form of individual guide beams 124B are aligned within each of the guide proximal ends 122P of the light guides 122A. The alignment assembly 256 can adjust the alignment and/or the positioning of the individual guide beams 124B relative to the receptacle assembly 274 and/or the light guides 122A. In some embodiments, the alignment assembly 256 can simultaneously adjust the positioning and/or alignment of the light guides 122A, the individual guide beams 124B, the multiplexer 128, and/or the receptacle assembly 274. The alignment assembly 256 can adjust the positioning of the guide proximal ends 122P of the light guides 122A so that the guide proximal ends 122P have one, two, or three degrees of freedom (rotational and/or translational). The alignment assembly 256 can adjust the positioning of the receptacle assembly 274 so that the receptacle assembly 274 has one, two, or three degrees of freedom (rotational and/or translational). The alignment assembly 256 can adjust the positioning of the receptacle assembly 274 relative to the multiplexer 128.

The alignment assembly 256 can vary depending on the design requirements of the catheter system 200, the type, size, and/or configuration of the multiplexer 128, the guide bundle 252, and/or the receptacle assembly 274. It is understood that the alignment assembly 256 can include additional components, systems, subsystems, and elements other than those specifically shown and/or described herein. Additionally, or alternatively, the alignment assembly 256 can omit one or more of the components, systems, subsystems, and elements that are specifically shown and/or described herein. In some embodiments, various components of the alignment assembly 256 can be positioned differently than what is specifically illustrated in Figure 2.

The alignment assembly 256 can include a static base 257, a first stage 258, a first stage knob 259, a second stage 260, a second stage knob 262, a rotary cam 264, and a mover 266. Additionally, as shown in the embodiment displayed in Figure 2, the alignment assembly 256 can be configured to engage and/or couple to the guide bundle 252 and/or the receptacle assembly 274. The alignment assembly 256 can be coupled to the multiplexer 128. In some embodiments, the alignment assembly 256 can include a camera 128c (for example, as illustrated in Figure 1). The camera 128c can capture images of the guide proximal ends 122P of each light guide 122A so that the position of the individual guide beams 124B relative to the guide proximal ends 122P can be adjusted.

The static base 257 can be configured as the base structure to which all the various components of the alignment assembly 256 are attached. For example, as illustrated in the embodiment shown in Figure 2, the second stage 260 can be affixed, coupled, attached, and/or otherwise engaged to the static base 257. The static base 257 can vary depending on the design requirements of the catheter system 200, the type, size, and/or configuration of the multiplexer 128, the guide bundle 252, the alignment assembly 256, and/or the receptacle assembly 274. It is understood that the static base 257 can include additional components, systems, subsystems, and elements other than those specifically shown and/or described herein. Additionally, or alternatively, the static base 257 can omit one or more of the components, systems, subsystems, and elements that are specifically shown and/or described herein. In some embodiments, various components of the static base 257 can be positioned differently than what is specifically illustrated in Figure 2.

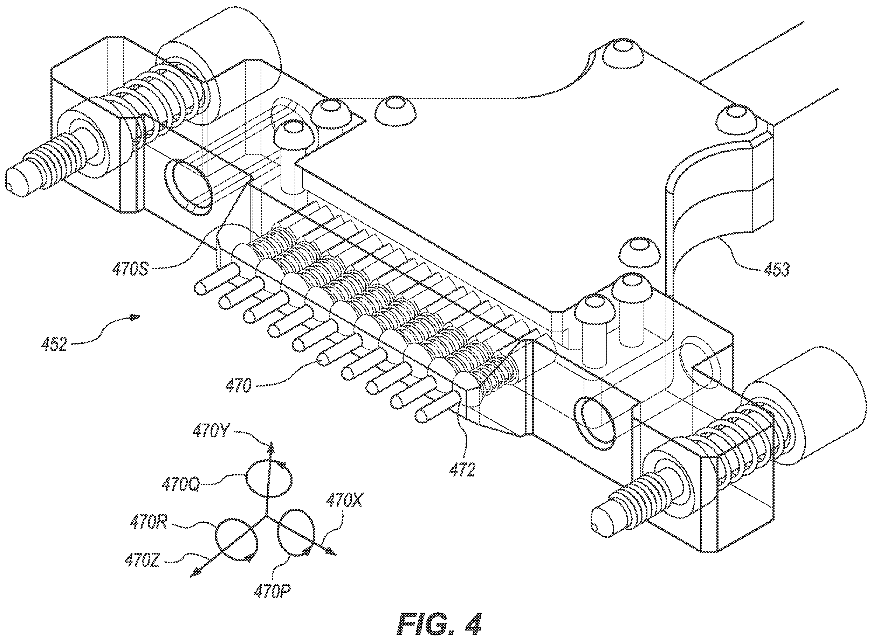

The first stage 258 can rotate about and/or move along any suitable axis. The first stage 258 can rotate about and/or move along a first axis 470X (illustrated in Figure 4), a second axis 470Y (illustrated in Figure 4), and a third axis 470Z (illustrated in Figure 4). The first stage 258 can rotate and/or move along any number of suitable axes as needed by the design requirements of the alignment assembly 256. The first stage 258 can be selectively fixed and/or coupled to the receptacle assembly 274.

In some embodiments, the first stage 258 is rotated by a system similar to a power train, where a first stage knob 259 is rotated by a mover 266. In other embodiments, the first stage 258 is coupled to a rotary cam 264 that controls the rotational motion of the first stage 258 about the light guide axis. In various embodiments, the first stage 258 can rotate on top of rollers 263 that are configured to move in cooperation with the rotational movement of the first stage 258. In some embodiments, the cams described herein (such as the rotary cam 264) can be replaced by linear movement rods or screws that move the knobs described herein (such as the first stage knob 259) in any suitable direction. The first stage knob 259 can selectively engage the rotary cam 264. The rotary cam 264 can be coupled to the mover 266.

The first stage 258 can vary depending on the design requirements of the catheter system 200, the type, size, and/or configuration of the multiplexer 128, the guide bundle 252, the alignment assembly 256, and/or the receptacle assembly 274. It is understood that the first stage 258 can include additional components, systems, subsystems, and elements other than those specifically shown and/or described herein. Additionally, or alternatively, the first stage 258 can omit one or more of the components, systems, subsystems, and elements that are specifically shown and/or described herein. In some embodiments, various components of the first stage 258 can be positioned differently than what is specifically illustrated in Figure 2.

The first stage 258 can be configured to receive and/or engage the receptacle assembly 274. In some embodiments, the first stage 258 will rotate the receptacle assembly 274 so that both components rotate in unison. In other embodiments, the first stage 258 can be integrally formed with the receptacle assembly 274.

The second stage 260 can lift and lower the first stage 258. In some embodiments, the second stage 260 can move the first stage 258 using a second stage knob 262. The second stage 260 can rotate about and/or move along any suitable axis. The second stage 260 can rotate about and/or move along a first axis 470X (illustrated in Figure 4), a second axis 470Y (illustrated in Figure 4), and a third axis 470Z (illustrated in Figure 4). The second stage 260 can rotate and/or move along any number of suitable axes as needed by the design requirements of the alignment assembly 256. In other embodiments, the second stage 260 can be configured to move the first stage 258 horizontally (along an x-axis). The second stage 260 can move about a second stage first axis (e.g., the second axis 470Y). The second stage 260 can be selectively fixed to the first stage 258.

The second stage 260 can vary depending on the design requirements of the catheter system 200, the type, size, and/or configuration of the multiplexer 128, the guide bundle 252, the alignment assembly 256, and/or the receptacle assembly 274. It is understood that the second stage 260 can include additional components, systems, subsystems, and elements other than those specifically shown and/or described herein. Additionally, or alternatively, the second stage 260 can omit one or more of the components, systems, subsystems, and elements that are specifically shown and/or described herein. In some embodiments, various components of the second stage 260 can be positioned differently than what is specifically illustrated in Figure 2.

The second stage 260 can be configured to receive and/or engage the first stage 258 and/or the receptacle assembly 274. In some embodiments, the second stage 260 will move in coordination with the first stage 258 and/or the receptacle assembly 274. In other embodiments, the second stage 260 can be integrally formed with the first stage 258 and/or the receptacle assembly 274.

The second stage knob 262 can be spring-loaded and can engage another cam (not shown). The second stage knob 262 can be driven by a mover 266. The rotary motion of the mover 266 can drive the second stage knob 262 to engage the rotary cam 264, which results in reciprocal motion of the second stage 260.

The second stage knob 262 can vary depending on the design requirements of the catheter system 200, the type, size, and/or configuration of the alignment assembly 256, the first stage 258, the second stage 260, and/or the receptacle assembly 274. It is understood that the second stage knob 262 can include additional components, systems, subsystems, and elements other than those specifically shown and/or described herein. Additionally, or alternatively, the second stage knob 262 can omit one or more of the components, systems, subsystems, and elements that are specifically shown and/or described herein. In some embodiments, various components of the second stage knob 262 can be positioned differently than what is specifically illustrated in Figure 2.

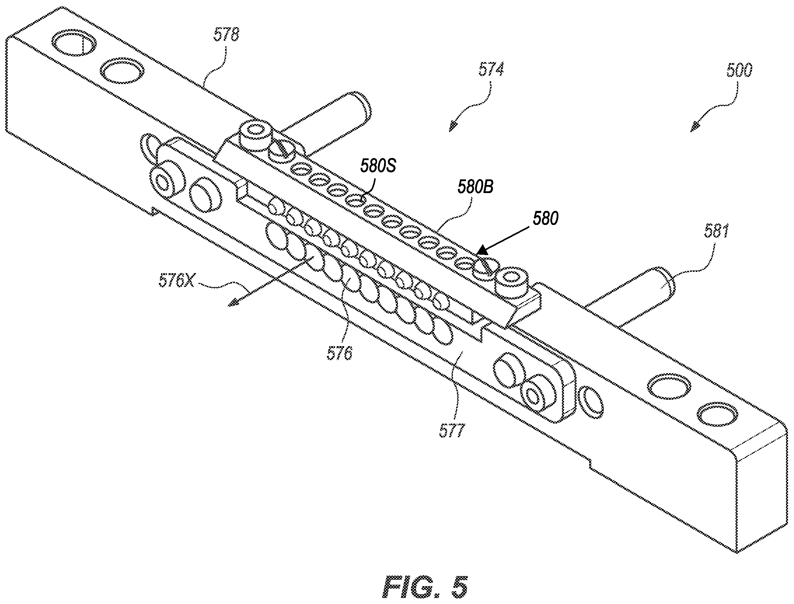

The receptacle assembly 274 can be configured to align the guide bundle ferrules 270 so that they are arranged in a linear array with a known pitch. In some embodiments, the receptacle assembly 274 can align and/or position the guide proximal ends 122P of the light guides 122A and/or the guide bundle ferrules 270 accurately within a margin of error of single-digit micrometers. The receptacle assembly 274 can receive the guide proximal ends 122P and/or the guide bundle ferrules 270 so that the guide proximal ends 122P and/or the guide bundle ferrules 270 are fixed along a receptacle ferrule receiver axis 576X (for example, as illustrated in Figure 5). The receptacle assembly 274 can be coupled to the alignment assembly 256.

The receptacle assembly 274 can vary depending on the design requirements of the catheter system 200, the type, size, and/or configuration of the multiplexer 128, the guide bundle 252, and/or the alignment assembly 256. It is understood that the receptacle assembly 274 can include additional components, systems, subsystems, and elements other than those specifically shown and/or described herein. Additionally, or alternatively, the receptacle assembly 274 can omit one or more of the components, systems, subsystems, and elements that are specifically shown and/or described herein. In some embodiments, various components of the receptacle assembly 274 can be positioned differently than what is specifically illustrated in Figure 2. Greater detail of the receptacle assembly 274 will be shown in Figure 5 and further described herein.

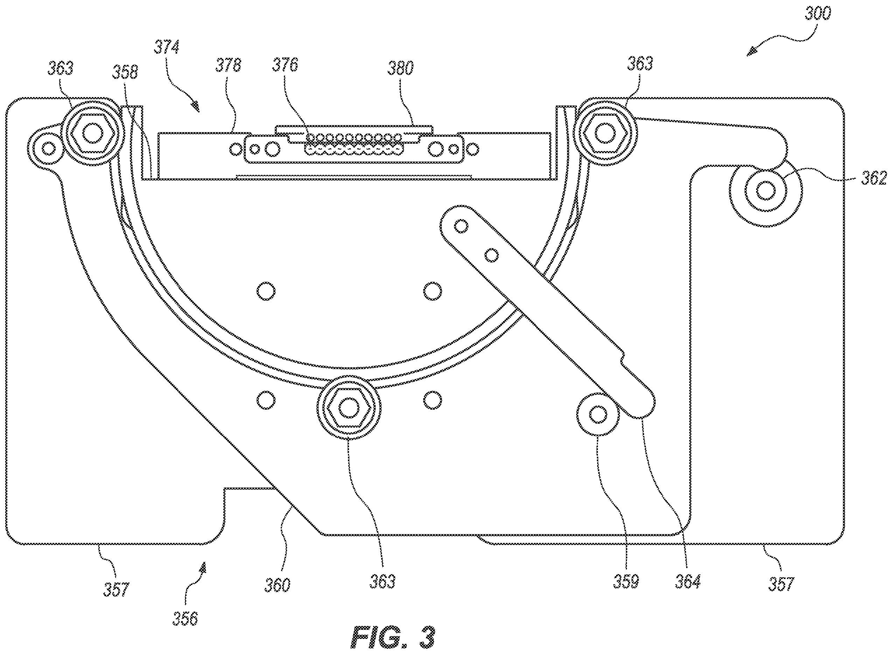

Figure 3 is a front view of a portion of an embodiment of a catheter system 300, including one embodiment of an alignment assembly 356 and a receptacle assembly 374. As illustrated in the embodiment displayed in Figure 3, the catheter system 300 can include the alignment assembly 356 (which can include a static base 357, a first stage 358, a first stage knob 359, a second stage 360, a second stage knob 362, a roller 363, and a rotary cam 364) and the receptacle assembly 374 (which can include a receptacle ferrule receiver 376, a receptacle assembly housing 378, and a receptacle ferrule retainer 380). The receptacle ferrule receiver 376, The receptacle assembly housing 378, and the receptacle ferrule retainer 380 will be described in further detail herein.

Figure 4 is a perspective view of a portion of an embodiment of the catheter system 100, including one embodiment of the guide bundle 452. As shown in the embodiment illustrated in Figure 4, the guide bundle 452 can include the guide bundle housing 453, guide bundle ferrules 470, and guide bundle apertures 472.

The guide bundle 452 bundles a multitude of light guides 122A (illustrated in Figure 1), each individually into a corresponding guide bundle ferrule 470. Each of the guide bundle ferrules 470 has at least one degree of freedom. In some embodiments, the guide bundle ferrules 470 have degrees of freedom about a first axis 470X, a second axis 470Y, a third axis 470Z, a first rotational axis 470P, a second rotational axis 470Q, a third rotational axis 470R, and/or any suitable axis. In various embodiments, any axis can be the first axis 470X, the second axis 470Y, and/or the third axis 470Z. In various embodiments, the axes described herein can be substantially orthogonal relative to one another, such as shown in Figure 4.

The guide bundle ferrules 470 can include springs 470S so that the guide bundle ferrules 470 are spring-loaded into the guide bundle 452 from which they protrude. In some embodiments, the springs 470S can provide a spring force to push the ferrules 470 along the first axis 470X, the second axis 470Y, and/or the third axis 470Z. The guide bundle apertures 472 through which the ferrules 470 protrude are large enough to allow small movements that are applied by the alignment correction mechanism that can be provided by the alignment assembly 256 (illustrated in Figure 2). The guide bundle ferrules 470 are each movable about the first axis 470X, the second axis 470Y, the third axis 470Z, the first rotational axis 470P, the second rotational axis 470Q, and/or the third rotational axis 470R. It is understood that the use of first, second, and third with respect to the axes and/or stages is merely for clarity and in some embodiments, the first, second, and third axes and/or stages are the same axes and/or stages. In other embodiments, the first, second, and third axes and/or stages are different axes and/or stages. In some embodiments, the first axis 470X, the second axis 470Y, and/or the third axis 470Z, are substantially orthogonal relative to one another.

Figure 5 is a perspective view of a portion of an embodiment of the catheter system 500, including one embodiment of the receptacle assembly 574. In some embodiments, the receptacle assembly 574 can include a receptacle ferrule receiver 576 having a receptacle ferrule receiver axis 576X, a receptacle block 577, a receptacle assembly housing 578, and a receptacle ferrule retainer 580.

The receptacle ferrule receiver 576 can receive an individual guide bundle ferrule 470 (illustrated in Figure 4). The receptacle ferrule receiver 576 can be formed into the receptacle block 577 of the receptacle assembly 574. A plurality of receptacle ferrule receivers 576 can be aligned into an array in any suitable distribution pattern (a linear pattern is displayed in Figure 5). Non-limiting, non-exclusive examples of distribution patterns include linear, circular, hexagonal, or any suitable geometric distribution pattern.

The receptacle ferrule receiver 576 can vary depending on the design requirements of the catheter system 500, the type, size, and/or configuration of the multiplexer 128 (illustrated in Figure 1), the guide bundle 252 (illustrated in Figure), and/or the alignment assembly 556. It is understood that the receptacle ferrule receiver 576 can include additional components, systems, subsystems, and elements other than those specifically shown and/or described herein. Additionally, or alternatively, the receptacle ferrule receiver 576 can omit one or more of the components, systems, subsystems, and elements that are specifically shown and/or described herein. In some embodiments, various components of the receptacle ferrule receiver 576 can be positioned differently than what is specifically illustrated in Figure 5. The receptacle ferrule receiver 576 can include an axial ferrule stopper.

The receptacle assembly housing 578 houses individual components of the receptacle assembly 574, such as the receptacle ferrule receiver 576, the receptacle block 577, and the receptacle ferrule retainer 580. The receptacle assembly housing 578 can enable the coupling of the receptacle assembly 574 to the multiplexer 128. The receptacle assembly housing 578 can include a guide pin 581 that guides the alignment, positioning, and/or coupling of the receptacle assembly housing 578 with the multiplexer 128. The receptacle assembly housing 578 can vary depending on the design requirements of the catheter system 500, the type, size, and/or configuration of the multiplexer 128 (illustrated in Figure 1), the guide bundle 252 (illustrated in Figure), and/or the receptacle assembly 574. In some embodiments, various components of the receptacle assembly housing 578 can be positioned differently than what is specifically illustrated in Figure 5. The receptacle assembly housing 578 can include a v-groove block.

The receptacle ferrule retainer 580 can selectively retain and/or lock any number of individual guide bundle ferrules 470 in desired locations within the receptacle ferrule receiver 576. The receptacle ferrule retainer 580 can retain the individual guide bundle ferrule 470 in one or more positions inside the receptacle ferrule receiver 576. The receptacle ferrule retainer 580 can vary depending on the design requirements of the catheter system 500, the type, size, and/or configuration of the multiplexer 128 (illustrated in Figure 1), the guide bundle 252 (illustrated in Figure), and/or the receptacle assembly 574. It is understood that the receptacle ferrule retainer 580 can include additional components, systems, subsystems, and elements other than those specifically shown and/or described herein.

Additionally, or alternatively, the receptacle ferrule retainer 580 can omit one or more of the components, systems, subsystems, and elements that are specifically shown and/or described herein. In some embodiments, various components of the receptacle ferrule retainer 580 can be positioned differently than what is specifically illustrated in Figure 5. The receptacle ferrule retainer 580 can include a plurality of spring-loaded plunger ball springs 580S that retain and/or lock corresponding individual guide bundle ferrules 470 into one or more positions. In some embodiments, the receptacle ferrule retainer 580 can include a plunger ball spring bridge 580B having plunger ball springs 580S that are each configured to selectively lock each of the guide bundle ferrules 470 in desired locations within the receptacle ferrule receiver 576.

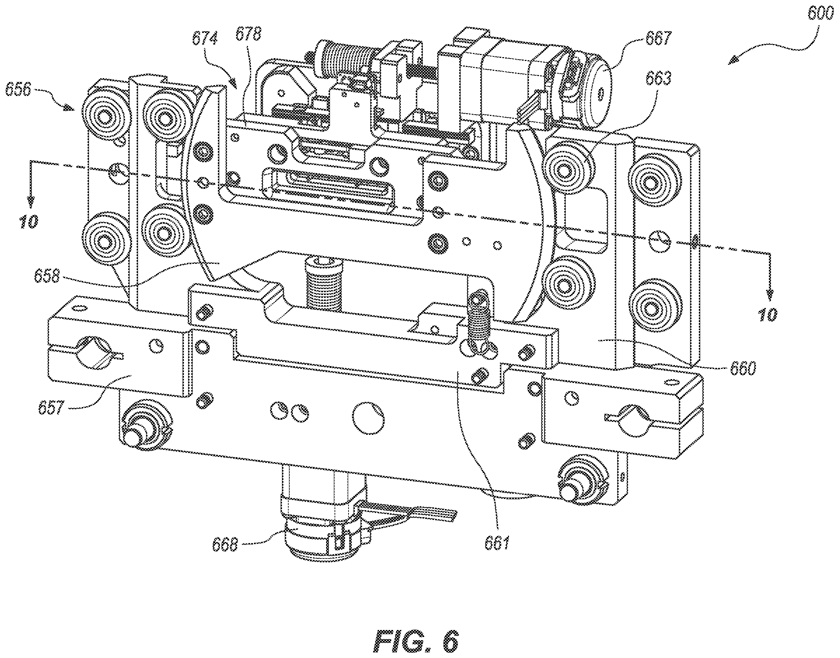

Figure 6 is a perspective view of a portion of an embodiment of the catheter system 600, including yet another embodiment of the alignment assembly 656 and the receptacle assembly 674. As illustrated in Figure 6, the alignment assembly 656 can include a static base 657, a first stage 658, a second stage 660, a third stage 661, a plurality of rollers 663, a first mover 667, and a second mover 668. The receptacle assembly 674 can include a receptacle assembly housing 678. The alignment assembly 656, the static base 657, the first stage 658, the second stage 660, the third stage 661, the plurality of rollers 663, the first mover 667, the second mover 668, the receptacle assembly 674, and the receptacle assembly housing 678 can be substantially similar to their counterparts described in other embodiments herein.

In some embodiments, the first stage 658, the second stage 660, and the third stage 661 can be separate stages. In other embodiments, the first stage 658, the second stage 660, and the third stage 661 can be the same stage or can be coupled to form one stage where the first stage 658, the second stage 660, and the third stage 661 move in cooperation. It is appreciated that the alignment assembly 656 can include any suitable number of stages to meet the design requirements of the catheter system 600 and/or the alignment assembly 656.

The plurality of rollers 663 are each configured to engage and/or roll one or more stages. For example, the rollers 663 can roll the first stage 658 along and/or about a suitable axis. The rollers 663 can include a roller bearing with a grooved mating face, a slotted wheel, a gear, and/or a pinion, as non-limiting, non-exclusive examples.

In certain embodiments, the first mover 667 and the second mover 668 are separate movers. In other embodiments, the first mover 667 and the second mover 668 can be the same mover or can be configured to move the same component. It is appreciated that the alignment assembly 656 can include any suitable number of movers to meet the design requirements of the catheter system 600 and/or the alignment assembly 656.

The first mover 667 and/or the second mover 668 can move the first stage 658, the second stage 660, and/or the third stage 661 along any suitable axis. In other embodiments, the first mover 667 and/or the second mover 668 can move the first stage 658, the second stage 660, and/or the third stage 661 about any suitable axis. Suitable axes include the first axis 470X (illustrated in Figure 4), the second axis 470Y (illustrated in Figure 4), the third axis 470Z (illustrated in Figure 4), the first rotational axis 470P (illustrated in Figure 4), the second rotational axis 470Q (illustrated in Figure 4) and the third rotational axis 470R (illustrated in Figure 4).

The first mover 667 and/or the second mover 668 can include a motor, an actuator, and/or a bearing. Bearings can be utilized within the movers in order to stabilize the movement provided by the movers.

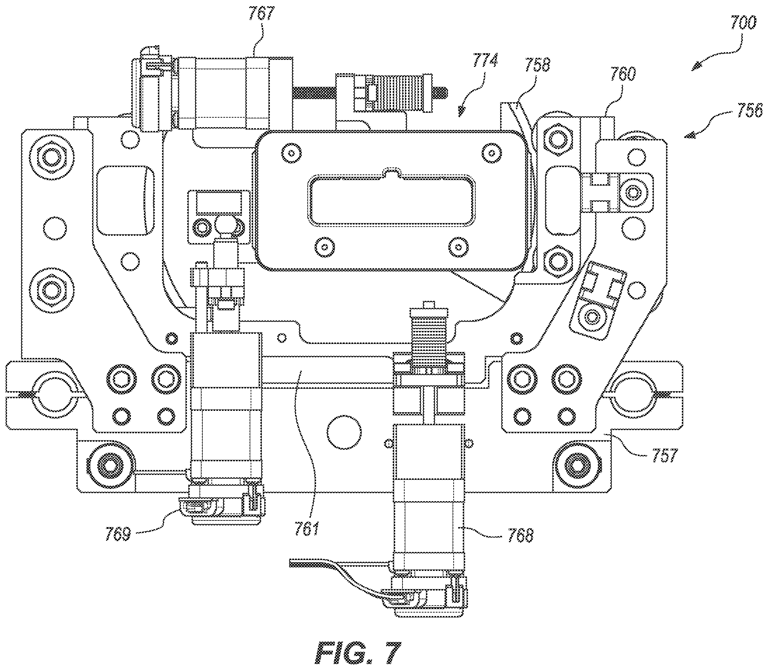

Figure 7 is a rear view of a portion of an embodiment of the catheter system 700, including one embodiment of the alignment assembly 756 and the receptacle assembly 774. As illustrated in Figure 7, the alignment assembly 756 can include a static base 757, a first stage 758, a second stage 760, a third stage 761, a first mover 767, a second mover 768, and/or a third mover 769. In the embodiment shown in Figure 7, the first mover 767 can move the first stage 758 about a first rotational axis 470P (illustrated in Figure 4). In other embodiments, the first mover 767 can move the receptacle assembly 774 about the first rotational axis 470P. The second mover 768 can move the second stage 760 along a first axis 470Z (illustrated in Figure 4). The third mover 769 can move the first stage 758 about a second rotational axis 470Q (illustrated in Figure 4).

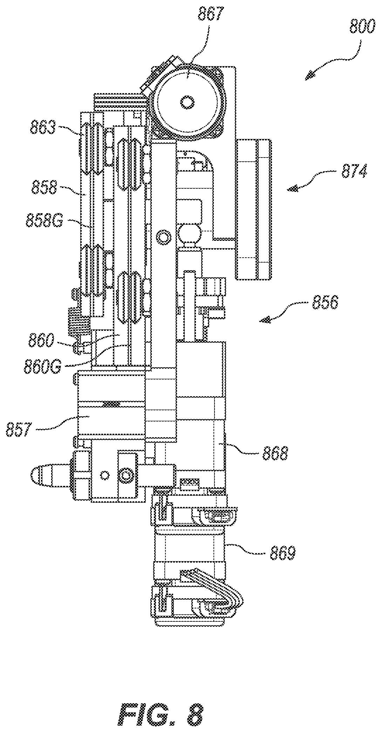

Figure 8 is a side view of a portion of an embodiment of the catheter system 800, including one embodiment of the alignment assembly 856 and the receptacle assembly 874. As illustrated in Figure 8, the alignment assembly 856 can include a static base 857, a first stage 858, a second stage 860, a plurality of rollers 863, a first mover 867, a second mover 868, and/or a third mover 869.

The first stage 858 can include a first stage guide 858G. The first stage guide 858G can guide the movement of the first stage 858. For example, in one non-exclusive, non-limiting embodiment, the first stage guide 858G includes a groove that engages a slot in a roller 863. The first stage guide 858G can guide the movement of the first stage 858 in any suitable direction, including a first axis 470X (illustrated in Figure 4), a second axis 470Y (illustrated in Figure 4), a third axis 470Z (illustrated in Figure 4), a first rotational axis 470P (illustrated in Figure 4), a second rotational axis 470Q (illustrated in Figure 4) and a third rotational axis 470R (illustrated in Figure 4).

The second stage 860 can include a second stage guide 860G that is substantially similar to the first stage guide 858G. The stages described herein can have any suitable number of stage guides. Non-limiting, non-exclusive examples of stage guides include grooves, tracks, ridges, seams, channels, and slits.

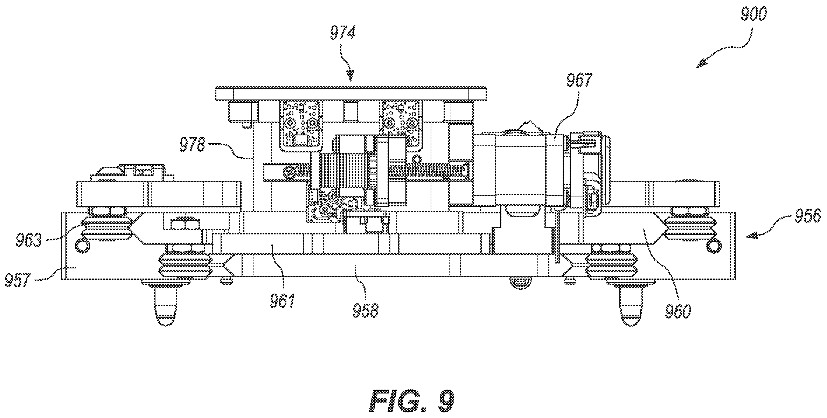

Figure 9 is a top view of a portion of an embodiment of the catheter system 900, including one embodiment of the alignment assembly 956 and the receptacle assembly 974. As illustrated in Figure 9, the alignment assembly 956 can include a static base 957, a first stage 958, a second stage 960, a third stage 961, a plurality of rollers 963, a first mover 967, and a second mover 968. The receptacle assembly 974 can include a receptacle assembly housing 978. The alignment assembly 956, the static base 957, the first stage 958, the second stage 960, the third stage 961, the plurality of rollers 963, the first mover 967, the second mover 968, the receptacle assembly 974, and the receptacle assembly housing 978 can be substantially similar to their counterparts described in other embodiments herein.

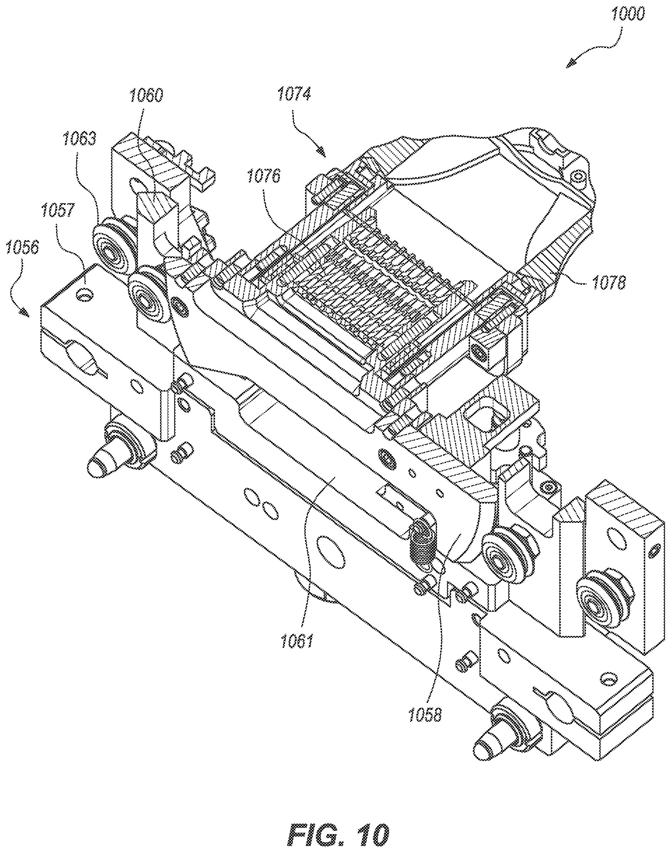

Figure 10 is a cross-sectional view of a portion of an embodiment of the catheter system 1000 taken on line 10-10 in Figure 6, including one embodiment of the alignment assembly 1056 and the receptacle assembly 1074. As illustrated in Figure 10, the alignment assembly 1056 can include a static base 1057, a first stage 1058, a second stage 1060, a third stage 1061, and a plurality of rollers 1063. The receptacle assembly 1074 can include a receptacle ferrule receiver 1076 and a receptacle assembly housing 1078. The alignment assembly 1056, the static base 1057, the first stage 1058, the second stage 1060, the third stage 1061, the plurality of rollers 1063, the receptacle assembly 1074, the receptacle ferrule receiver 1076, and the receptacle assembly housing 1078 can be substantially similar to their counterparts described in other embodiments herein.

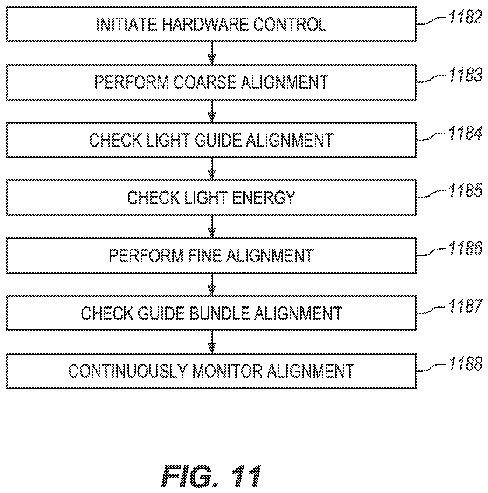

Figure 11 is a flow chart depicting one embodiment of a method for aligning a light source within a catheter system 100 (illustrated in Figure 1). It is understood that the method can include additional steps than those specifically shown and/or described herein. Additionally, or alternatively, the method can omit one or more of the steps that are specifically shown and/or described herein. The method for alignment can be implemented on the catheter system 100 or other systems and subsystems not specifically shown and/or described herein.

At step 1182, the alignment hardware is initialized. As used herein, "alignment hardware" can include (as non-limiting, non-exclusive examples) cameras 128c (illustrated in Figure 1), illuminating devices, the guide bundle 152 (for example, illustrated in Figure 1), the alignment assembly 256 (illustrated in Figure 2), the static base 257 (illustrated in Figure 2), the first stage 258 (illustrated in Figure 2), the second stage 260 (illustrated in Figure 2), the second stage knob 262 (illustrated in Figure 2), the rollers 263 (illustrated in Figure 2), the rotary cam 264 (illustrated in Figure 2), one or more movers 266 (illustrated in Figure 2), the receptacle assembly 374 (illustrated in Figure 3), the receptacle ferrule receiver 376 (illustrated in Figure 3), the receptacle assembly housing 378 (illustrated in Figure 3), and/or the receptacle ferrule retainer 380 (illustrated in Figure 3).

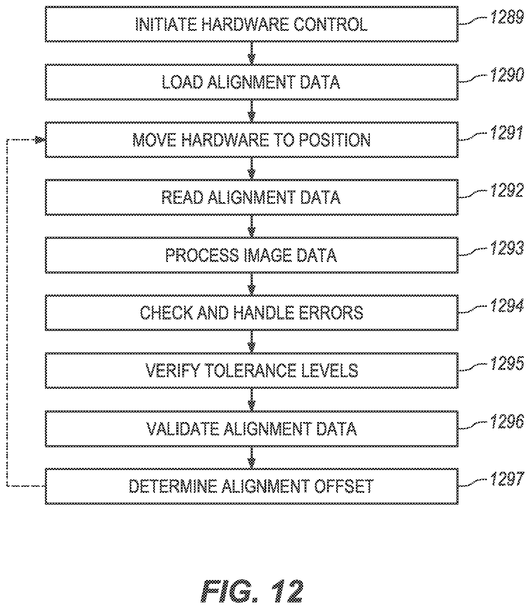

At step 1183, a coarse alignment of the alignment hardware is performed. The coarse alignment can be performed in any suitable manner. One non-limiting, non-exclusive example of a method for performing the coarse alignment is displayed in Figure 12 and described in greater detail herein. The coarse alignment of the alignment hardware can include adjusting the x-position, height (y-position), z-position, tilt (pitch), roll, yaw, and/or any suitable position along or about any suitable axis of the alignment hardware using one or more movers and/or adjusters.

At step 1184, the light guide alignment with the light source is checked. In some embodiments, the alignment of the light guide with the light source can be checked using a camera and an illuminating source (such as a light-emitting diode). The camera can capture images of the proximal end of the light guide. The system controller can generate an X-Y-Z reference plane of coordinates using the images captured by the camera.

The system controller can detect the features of the proximal end of the light guide using one or more reference features from a reference image. The system controller can identify pixels within the captured image. In some embodiments, the system controller can detect features with sub-pixel pitch accuracy. Non-limiting, non-exclusive examples of features and reference features include end faces of light guides, light guide apertures, light guide receivers, light guide energy dumps, light guide targets, and/or light guide cores. The reference features from the reference images can include a wide range of variations in images, including details such as exposure levels, image resolution, lighting variation, camera defects and artifacts, feature sizes, and/or image qualities.

The system controller can calculate a tolerance region for each feature compared to the reference features. The system controller can then verify that the alignment is within the desired tolerance level using the calculated tolerance region.

At step 1185, the light energy of the light source is checked. In some embodiments, a laser is the light source, and the power levels of the laser are checked.

At step 1186, a fine alignment of the alignment hardware is performed. The fine alignment can be performed in any suitable manner. In some embodiments, the fine alignment can be performed somewhat similarly to the coarse alignment.

At step 1187, the guide bundle alignment is checked. In some embodiments, the guide pins of the alignment assembly are checked to ensure proper alignment with the guide bundle.

At step 1188, the alignment of the light guide with the light source is continuously monitored until the completion of the treatment. In some embodiments, the continuous monitoring of the alignment of the light guide with the light source can end upon the completion of the method for aligning the light source within the catheter system.

Figure 12 is a flow chart depicting one embodiment of a method for performing a coarse alignment of a light source within a catheter system in accordance with various embodiments herein. It is understood that the method can include additional steps than those specifically shown and/or described herein. Additionally, or alternatively, the method can omit one or more of the steps that are specifically shown and/or described herein. The method for alignment can be implemented on the catheter system or other systems and subsystems not specifically shown and/or described herein.

At step 1289, control of the alignment hardware is initialized. The control initialization can include detection of the light guide bundle and/or connector, checking one or more system modules, powering up the system, setting up one or more cameras, setting up one or more illuminators, powering on one or more stages, and/or alignment hardware.

At step 1290, alignment data is loaded. The loaded alignment data can include one or more of hardcoded settings, calibration settings, parameters, alignment offsets, control settings, error codes, and/or settings for an image path. In some embodiments, the loaded alignment data can also include a plurality of light guide reference locations and/or a plurality of coordinates in a reference plane. The reference plane can include a 3-D grid having a first axis, a second axis, and a third axis. In some embodiments, the suitable axis within the reference plane includes an x-axis, a y-axis, and a z-axis. The plurality of light guide reference locations can include light guide end face reference locations.

At step 1291, the alignment hardware is moved to a position. The positioning can include homing to a first stage. The step of positioning can include importing of data dictionaries and performing system error checks.

At step 1292, alignment data is read. The camera can be utilized as machine vision for detecting a first picture, once the alignment hardware is moved to a first position. One or more cameras can capture images taken at the first position. The cameras can continue to capture images until all the reference targets are captured. If the targets are not fully captured, the stage can be moved to a second position, a third position, or any suitable number of positions until all reference targets are captured by the one or more cameras.

At step 1293, image data is processed. Once all reference targets have been captured by one or more cameras, the system detects the reference features. One or more image processing algorithms can process the image data. The image processing algorithms can utilize feature detection, such as described herein. The image processing algorithms can cooperate with one or more evaluation matrices to improve the algorithms by training with a wide range of reference image data, including exposure levels, image resolution, lighting variation, camera defects, and artifacts, feature sizes, and/or image qualities. In some embodiments, the evaluation matrices can include over 600 hundred datasets containing more than 10,000 images.

At step 1294, errors are checked and handled by the system. If errors are detected within the system, the system is updated, and the errors are handled by error handling. If no errors are detected within the system, the method continues to step 1295.

At step 1295, tolerance levels of the alignment are verified. The verification can include calculating one or more tolerance regions for each reference target of a feature. After the tolerance regions are calculated, the alignment is verified to determine it is within the one or more tolerance regions.

At step 1296, the alignment data is validated. One or more alignment coordinates can be validated at this step. The processing of the alignment data can include filtering, averaging, statistic generation, and any other suitable data processing. If there is a data validation error, the system is updated, and the errors are handled by error handling. Data validation errors can include errors within the system, false negatives, false positives, and errors in feature and/or reference detection.

At step 1297, the alignment offset is determined. The alignment offset can be adjusted at this step. The adjustments can include removing the camera offset. A linear regression can be performed to calculate one or more axis offset values. The one or more axis offset values can include an x-axis offset, a y-axis offset, a z-axis offset, a pitch offset, a tilt offset, a yaw offset, and/or a roll offset. If the coarse alignment is verified at step 1297, the method for coarse alignment ends. If the coarse alignment is not within the acceptable ranges, the method repeats steps 1291 through 1296. After completion of the determination of the offsets, the tolerance levels can be checked for each individual feature. In some embodiments, the alignment adjustment can be verified with three or more rechecks.

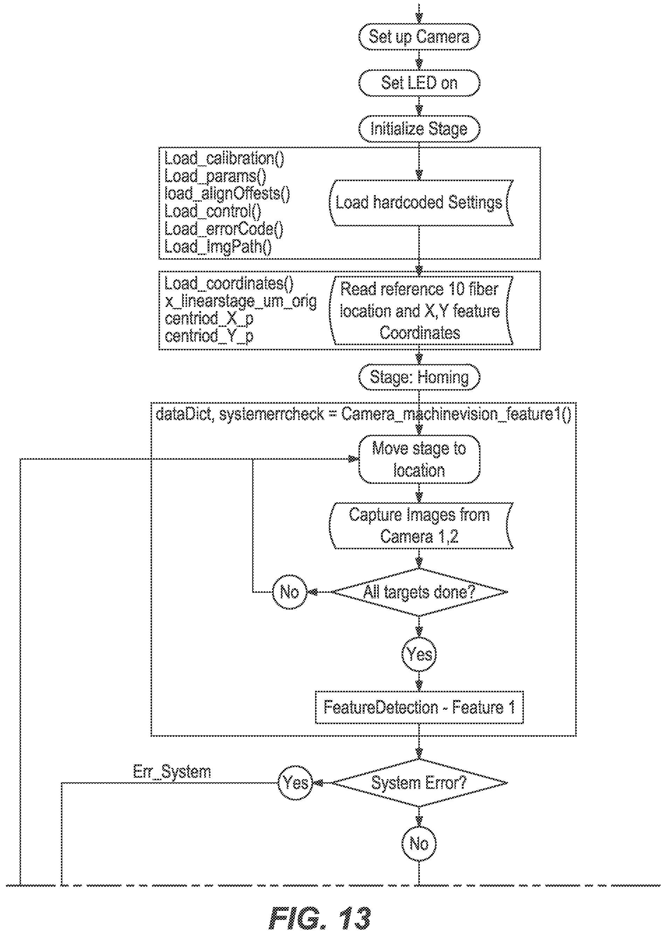

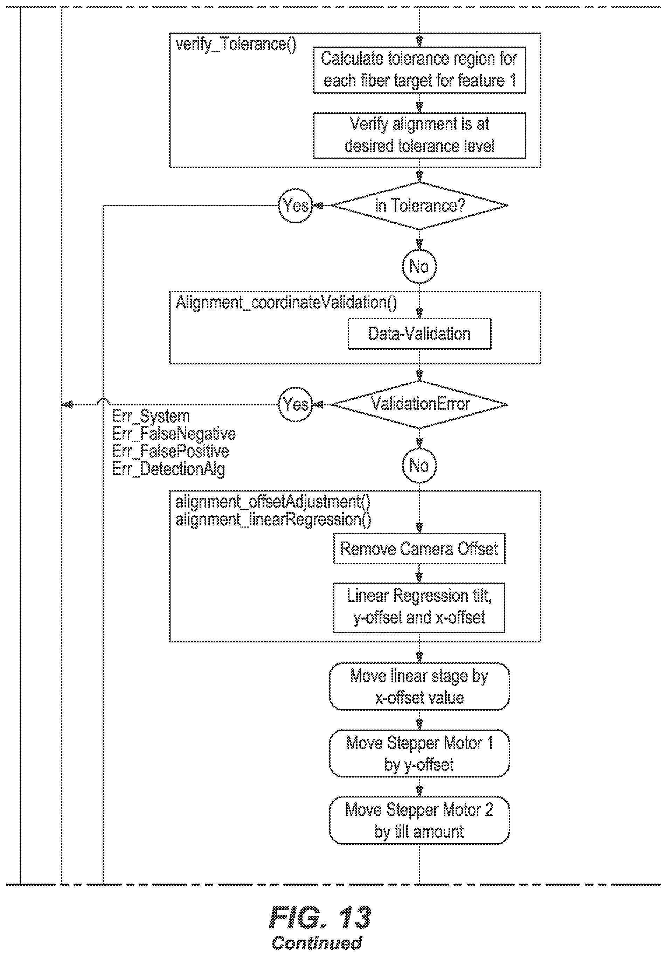

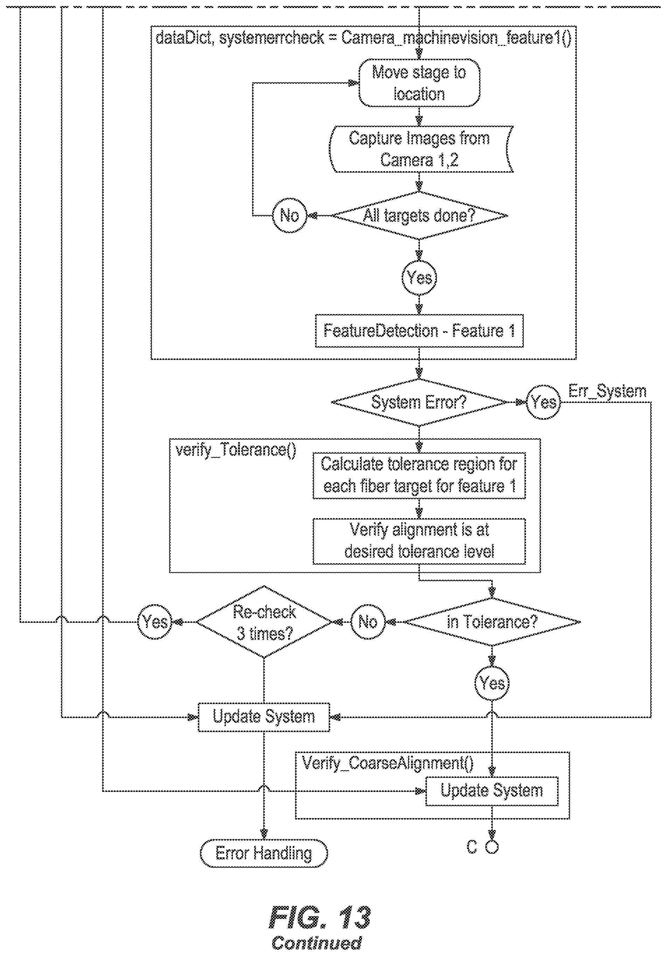

Figure 13 is a flow chart depicting yet another embodiment of a method for performing a coarse alignment of a light source 124 (illustrated in Figure 1) within a catheter system (illustrated in Figure 1) in accordance with various embodiments herein. It is understood that the method can include additional steps than those specifically shown and/or described herein. Additionally, or alternatively, the method can omit one or more of the steps that are specifically shown and/or described herein. The method in Figure 13 can be substantially similar to the method shown and described with respect to Figure 12, but can also include additional steps and substeps, as shown.

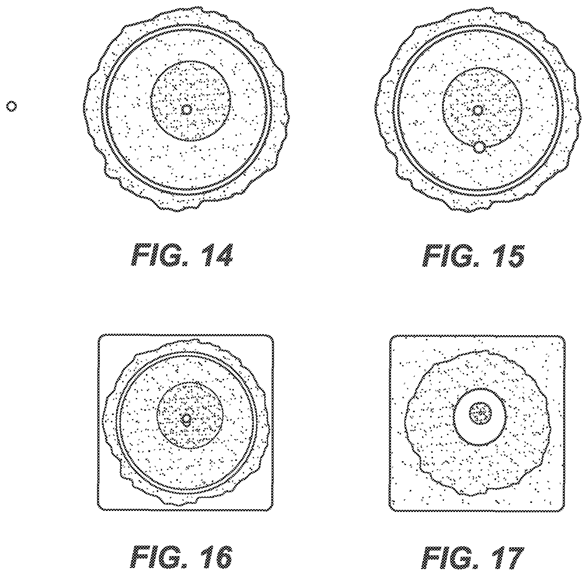

Figure 14 is an illustration of an end face of a light guide 122A (illustrated in Figure 1) captured by a camera 128c (illustrated in Figure 1) during one embodiment of a method for aligning a light source within a catheter system.

Figure 15 is another illustration of an end face of a light guide 122A (illustrated in Figure 1) captured by a camera 128c (illustrated in Figure 1) during one embodiment of a method for aligning a light source within a catheter system.

Figure 16 is yet another illustration of an end face of a light guide 122A (illustrated in Figure 1) captured by a camera 128c (illustrated in Figure 1) during one embodiment of a method for aligning a light source within a catheter system.

Figure 17 is yet another illustration of an end face of a light guide 122A (illustrated in Figure 1) captured by a camera 128c (illustrated in Figure 1) during one embodiment of a method for aligning a light source within a catheter system.

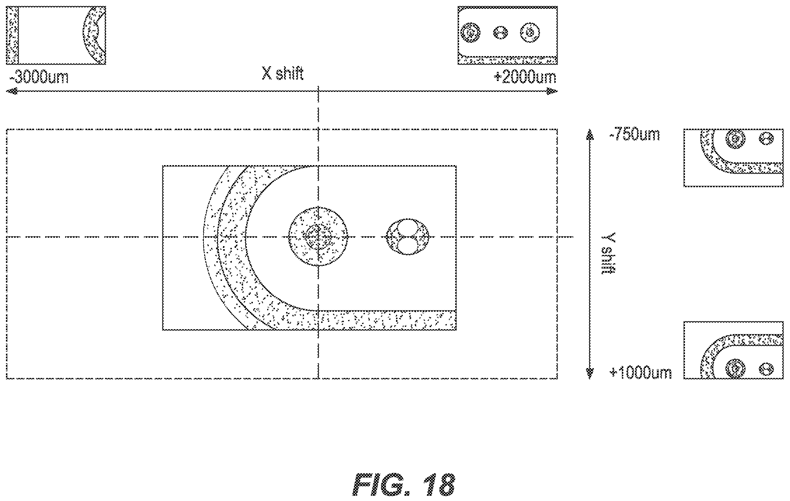

Figure 18 is an illustration of a user interface displaying an overlaid coordinate plane used during one embodiment of a method for aligning a light source within a catheter system.

As described in detail herein, in various embodiments, the alignment assembly and receptacle assemblies can be utilized to solve many problems that exist in more traditional catheter systems. For example:
1) In some embodiments, the present technology allows the use of low-cost components with a low resolution of movement, low accuracy, and tolerance of mechanical dimensions and movement to translate into micrometer level corrections of the positioning of the line connecting the light guides in the light guide array. This is achieved by using adjusters and/or movers, including knobs and cams far enough from the location of the light guides that are the subject of positional correction.
2) The alignment and receptacle assemblies can be implemented in any suitable direction(s). In some embodiments, the axial direction (of the light guides and source beam) determines the alignment of the placement of the connector with the line of focus for the beam, and such a mechanism can be moved in synchronization with the multiplexer/beam scanner such that each light guide will be positioned in the desired focal length from the coupling optics.

It should be noted that, as used in this specification and the appended claims, the singular forms "a," "an," and "the" include plural referents unless the content and/or context clearly dictates otherwise. It should also be noted that the term "or" is generally employed in its sense, including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the spirit and scope herein.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions, and sub-combinations thereof. It is therefore intended that the following appended claims and claims hereafter introduced are interpreted to include all such modifications, permutations, additions, and sub-combinations as are within their true spirit and scope, and no limitations are intended to the details of construction or design herein shown.

The following aspects are preferred embodiments of the present invention.
1. A catheter system for treating a vascular lesion within or adjacent to a vessel wall within a body of a patient, the catheter system comprising:
   a light source that generates a source beam of light energy;
   a receptacle assembly;
   a first light guide and a second light guide that is coupled to the receptacle assembly, each light guide having a guide proximal end;
   a multiplexer that receives the source beam from the light source, the multiplexer directing individual guide beams from the source beam to each of the guide proximal end of the first light guide and the guide proximal end of the second light guide; and
   an alignment assembly that adjusts the position of the receptacle assembly relative to the individual guide beams.
2. The catheter system of aspect 1 wherein each of the guide proximal end of the first light guide and the guide proximal end of the second light guide has two rotational degrees of freedom.
3. The catheter system of any of aspects 1-2 wherein the receptacle assembly has three degrees of freedom.
4. The catheter system of any of aspects 1-3 wherein the receptacle assembly has three rotational degrees of freedom.
5. The catheter system of any of aspects 1-4 wherein the multiplexer has at least one degree of freedom.
6. The catheter system of any of aspects 1-5 wherein the alignment assembly adjusts the receptacle assembly relative to the individual guide beams in micrometer level adjustments.
7. The catheter system of any of aspects 1-6 wherein the alignment assembly adjusts the receptacle assembly relative to the multiplexer.
8. The catheter system of any of aspects 1-7 wherein the receptacle assembly is coupled to the alignment assembly.
9. The catheter system of any of aspects 1-8 wherein the multiplexer is coupled to the alignment assembly.
10. The catheter system of any of aspects 1-9 wherein the alignment assembly includes a camera that captures images of the guide proximal ends of each light guide so that an alignment of the individual guide beams relative to the guide proximal ends can be adjusted.
11. A catheter system for treating a vascular lesion within or adjacent to a vessel wall within a body of a patient, the catheter system including a light source that generates a source beam of light energy, a receptacle assembly, a first light guide and a second light guide that are coupled to the receptacle assembly, each light guide having a guide proximal end, a multiplexer that receives the source beam from the light source, the multiplexer directing individual guide beams from the source beam to each of the guide proximal end of the first light guide and the guide proximal end of the second light guide, the catheter system comprising:
   an alignment assembly that adjusts the position of the receptacle assembly relative to the individual guide beams.
12. The catheter system of aspect 11 wherein the alignment assembly adjusts the receptacle assembly relative to the individual guide beams in micrometer level corrections.
13. The catheter system of any of aspects 11-12 wherein the alignment assembly adjusts the receptacle assembly relative to the multiplexer.
14. The catheter system of any of aspects 11-13 wherein the receptacle assembly is coupled to the alignment assembly.
15. The catheter system of any of aspects 11-14 wherein the multiplexer is coupled to the alignment assembly.
16. The catheter system of any of aspects 11-15 wherein each of the guide proximal ends is coupled to the alignment assembly so that the guide proximal ends have two rotational degrees of freedom.
17. The catheter system of any of aspects 11-16 wherein the receptacle assembly is coupled to the alignment assembly so that the receptacle assembly has three degrees of freedom.
18. The catheter system of any of aspects 11-17 wherein the receptacle assembly is coupled to the alignment assembly so that the receptacle assembly has three rotational degrees of freedom.
19. The catheter system of any of aspects 11-18 wherein the multiplexer is coupled to the alignment assembly so that the multiplexer has at least one degree of freedom.
20. The catheter system of any of aspects 11-19 wherein the alignment assembly includes a camera that captures images of the guide proximal ends of each light guide so that an alignment of the individual guide beams relative to the guide proximal ends can be adjusted.
21. The catheter system of any of aspects 11-20 further comprising a system controller including a processor that is configured to control operation of the light source to generate a single source beam in the form of pulses of light energy that are directed to the multiplexer; the system controller being further configured to control operation of the multiplexer so that a first guide beam is directed to the first light guide and a second guide beam is directed to the second light guide.
22. The catheter system of any of aspects 11-21 wherein the light source includes a laser.
23. The catheter system of any of aspects 11-22 further comprising a catheter shaft and a balloon that is coupled to the catheter shaft, the balloon including a balloon wall that defines a balloon interior, the balloon being configured to retain a balloon fluid within the balloon interior; wherein the first light guide and the second light guide are positioned at least partially within the balloon interior.
24. The catheter system of aspect 23 wherein the balloon is selectively inflatable with the balloon fluid to expand to an inflated state, wherein when the balloon is in the inflated state the balloon wall is configured to be positioned substantially adjacent to the vascular lesion.
25. The catheter system of aspect 24 wherein the first light guide and the second light guide receive the light energy from the light source and guide the light energy from the light source into the balloon interior to generate plasma in the balloon fluid within the balloon interior, the plasma generation causing rapid bubble formation and imparting pressure waves upon the balloon wall adjacent to the vascular lesion.
26. The catheter system of any of aspects 11-25 wherein the multiplexer includes an optical element that splits the source beam into a first guide beam and a second guide beam.
27. The catheter system of aspect 26 wherein the multiplexer further includes coupling optics that are configured to focus the first guide beam onto the first light guide and the second guide beam onto the second light guide.
28. The catheter system of aspect 27 wherein the first guide beam and the second guide beam are incident on the coupling optics with an angle between them.
29. The catheter system of any of aspects 11-28 further comprising a guide bundle that brings each of the first light guide and the second light guide closer together so that the first light guide and the second light guide are in a more compact form.
30. The catheter system of any of aspects 11-29 wherein the receptacle assembly includes a receptacle assembly housing, a receptacle ferrule receiver, and a receptacle ferrule retainer.
31. The catheter system of any of aspects 11-30 wherein the receptacle ferrule retainer selectively locks each of the first light guide and the second light guide in desired locations within the receptacle ferrule receiver.
32. The catheter system of any of aspects 30-31 wherein the receptacle ferrule retainer is a plunger ball spring bridge including a plurality of plunger ball springs that are each configured to selectively lock each of the first light guide and the second light guide in desired locations within the receptacle ferrule receiver.
33. The catheter system of any of aspects 11-32 wherein the alignment assembly includes a first stage selectively fixed to the receptacle assembly, the first stage being configured to rotate about one of a first axis and a second axis.
32. The catheter system of aspect 33 wherein the alignment assembly further includes a first stage knob that selectively engages a first rotary cam.
34. The catheter system of aspect 32 wherein the first rotary cam is coupled to a first mover.
35. The catheter system of aspect 34 wherein the first mover drives the first rotary cam so that the first stage moves in a rotary motion.
36. The catheter system of any of aspects 34-35 wherein the alignment assembly includes a second stage selectively fixed to the first stage, the second stage being configured to lift and lower the first stage on a first axis.
37. The catheter system of any of aspects 33-36 wherein the alignment assembly includes a second stage knob that engages a second rotary cam.
38. The catheter system of aspect 37 wherein the second rotary cam is coupled to a second mover.
39. The catheter system of aspect 38 wherein the second mover drives the second rotary cam so that the second stage lifts and lowers the first stage on a first axis.
40. A method for aligning a light source within a catheter system for treating a vascular lesion within or adjacent to a vessel wall within a body of a patient, the catheter system including the single light source that generates light energy, the method comprising the steps of:
   initiating control of a plurality of hardware of the catheter system, the plurality of hardware including at least one of (i) a light guide that is configured to selectively receive light energy from the light source, (ii) a camera that captures an alignment of the light source within the light guide, (iii) a mover that is configured to selectively adjust a positioning of the light source within the light guide;
   performing a coarse alignment of the positioning of the light source within the light guide;
   checking at least one of (i) the alignment of the light source within the light guide, and (ii) a light energy level of the light source; and
   performing a fine alignment of the positioning of the light source within the light guide.
41. The method of aspect 42 further comprising the step of checking a light connector alignment.
42. The method of any of aspects 40-41 further comprising the step of continuously monitoring the alignment of the light source within the light guide with the camera.
43. The method of any of aspects 40-42 wherein the step of performing the coarse alignment includes loading hardcoded alignment settings of an alignment algorithm that is configured to autonomously align the light source with the light guide.
44. The method of aspect 43 wherein the step of performing the coarse alignment includes loading a plurality of light guide reference locations and coordinates from the alignment algorithm.
45. The method of any of aspects 40-44 wherein the step of performing the coarse alignment includes initializing at least one of (i) the camera, (ii) an illumination source that is configured to illuminate an end face of the light guide, and (iii) a stage that is coupled to the light guide, the stage being configured to adjust the positioning of the light guide relative to the light source.
46. The method of aspect 45 wherein the step of performing the coarse alignment includes moving the stage to a second stage location.
47. The method of aspect 46 wherein the step of performing the coarse alignment includes capturing a plurality of captured images with the camera while the stage is in the second stage location.
48. The method of aspect 47 wherein the step of performing the coarse alignment includes determining if a plurality of reference targets are included in the plurality of captured images.
49. The method of aspect 48 wherein if the plurality of reference targets are included in the plurality of captured images, the step of performing the coarse alignment includes detecting a plurality of light guide reference locations and coordinates with an alignment algorithm that is configured to autonomously align the light source with the light guide.
50. The method of aspect 48 wherein if the plurality of reference targets are not included in the plurality of captured images, the step of performing the coarse alignment includes moving the stage to a second stage location and capturing a plurality of second captured images with the camera while the stage is in the second stage location.
51. The method of any of aspects 40-50 wherein the step of performing the coarse alignment includes checking the catheter system for system errors.
52. The method of aspect 51 wherein if the catheter system has one or more system errors, the catheter system is updated, and error handling is initiated.
53. The method of aspect 51 wherein if the catheter system does not have has one or more system errors, the step of performing the coarse alignment includes calculating a first tolerance region for a plurality of light guide reference locations and coordinates.
54. The method of aspect 53 wherein the step of performing the coarse alignment includes verifying that the alignment of the light source within the light guide is within the first tolerance region for the plurality of light guide reference locations and coordinates.
55. The method of aspect 54 wherein if the alignment of the light source within the light guide is within the first tolerance region for the plurality of light guide reference locations and coordinates, the step of performing the coarse alignment is completed.
56. The method of aspect 54 wherein if the alignment of the light source within the light guide is not within the first tolerance region for the plurality of light guide reference locations and coordinates, the step of performing the coarse alignment includes validating a plurality of alignment data.
57. The method of aspect 56 wherein if the validating of the plurality of alignment data results in a validation error, the catheter system is updated, and error handling is initiated.
58. The method of aspect 56 wherein if the validating of the plurality of alignment data does not result in a validation error, the step of performing the coarse alignment further includes removing a plurality of camera offsets; and performing a linear regression with respect to a plurality of hardware offsets.
59. The method of aspect 58 wherein the plurality of hardware offsets include at least one of a tilt offset, a y-axis offset, and an x-axis offset.
60. The method of aspect 58 wherein the step of performing the coarse alignment further includes positioning the plurality of hardware based on the plurality of hardware offsets.
61. The method of aspect 58 wherein the step of performing the coarse alignment further includes moving the stage to a third stage location and capturing a plurality of third captured images with the camera while the stage is in the third stage location.
62. A catheter system for treating a vascular lesion within or adjacent to a vessel wall within a body of a patient, the catheter system comprising:
   a light source that generates a source beam of light energy;
   a receptacle assembly;
   a first light guide and a second light guide that is coupled to the receptacle assembly, each light guide having a guide proximal end;
   a multiplexer that receives the source beam from the light source, the multiplexer directing individual guide beams from the source beam to each of the guide proximal end of the first light guide and the guide proximal end of the second light guide; and
   an alignment assembly that adjusts the position of the receptacle assembly relative to the individual guide beams, the alignment assembly including a first mover that moves the receptacle assembly.
63. The catheter system of aspect 62 wherein the first mover includes at least one of a motor and an actuator.
64. The catheter system of aspect 62 wherein the first mover includes a rack and pinion motor.
65. The catheter system of any of aspects 62-64 wherein the alignment assembly further includes a plurality of rollers coupled to the first mover, the plurality of rollers being configured to roll the first stage about the first axis.
66. The catheter system of any of aspects 62-65 wherein the alignment assembly includes a mechanical flexure that cooperates with the first mover to create angular displacement.
67. The catheter system of any of aspects 62-65 wherein the alignment assembly includes a mechanical flexure that cooperates with the first mover to create linear displacement.
68. The catheter system of aspect 62 wherein the first mover includes a screw mechanism and a motor.
69. The catheter system of aspect 62 wherein the first mover includes one of a piezoelectric stack and an actuator.

## Claims

1. A method for aligning a light source (124) within a catheter system (100) for treating a vascular lesion (106A) within or adjacent to a vessel wall (108A) within a body (107) of a patient (109), the catheter system (100) including the single light source (124) that generates light energy, the method comprising the steps of:
initiating control of a plurality of hardware of the catheter system (100), the plurality of hardware including at least one of (i) a light guide (122A) that is configured to selectively receive light energy from the light source (124), (ii) a camera (128C) that captures an alignment of the light source (124) within the light guide (122A), (iii) a mover (266, 667, 668) that is configured to selectively adjust a positioning of the light source (124) within the light guide (122A);
performing a coarse alignment of the positioning of the light source (124) within the light guide (122A);
checking at least one of (i) the alignment of the light source (124) within the light guide (122A), and (ii) a light energy level of the light source (124); and
performing a fine alignment of the positioning of the light source (124) within the light guide (122A).

2. The method of claim 1, further comprising the step of checking a light connector alignment.

3. The method of any one of claims 1-2, further comprising the step of continuously monitoring the alignment of the light source (124) within the light guide (122A) with the camera (128C).

4. The method of any one of claims 1-3, wherein the step of performing the coarse alignment includes loading hardcoded alignment settings of an alignment algorithm that is configured to autonomously align the light source (124) with the light guide (122A).

5. The method of claim 4, wherein the step of performing the coarse alignment includes loading a plurality of light guide reference locations and coordinates from the alignment algorithm.

6. The method of any one of claims 1-5, wherein the step of performing the coarse alignment includes initializing at least one of (i) the camera (128C), (ii) an illumination source that is configured to illuminate an end face of the light guide (122A), and (iii) a stage (258, 260) that is coupled to the light guide (122A), the stage (258, 260) being configured to adjust the positioning of the light guide (122A) relative to the light source (124).

7. The method of claim 6, wherein the step of performing the coarse alignment includes moving the stage (258, 260) to a second stage location and capturing a plurality of captured images with the camera (128C) while the stage (258, 260) is in the second stage location.

8. The method of claim 7, wherein the step of performing the coarse alignment includes determining if a plurality of reference targets are included in the plurality of captured images, preferably further including:
if the plurality of reference targets are included, detecting a plurality of light guide reference locations and coordinates with an alignment algorithm that is configured to autonomously align the light source with the light guide, and
if the plurality of reference targets are not included, moving the stage (258, 260) to a further stage location and capturing a plurality of second captured images with the camera (128C).

9. The method of any one of claims 1-8, wherein the step of performing the coarse alignment includes checking the catheter system (100) for system errors, and wherein if one or more system errors are present, the catheter system (100) is updated and error handling is initiated.

10. The method of claim 9, wherein if the catheter system (100) does not have one or more system errors, the step of performing the coarse alignment includes calculating a first tolerance region for a plurality of light guide reference locations and coordinates and verifying that the alignment of the light source (124) within the light guide (122A) is within the first tolerance region.

11. The method of claim 10, wherein if the alignment of the light source (124) within the light guide (122A) is within the first tolerance region, the step of performing the coarse alignment is completed, and if the alignment of the light source (124) within the light guide (122A) is not within the first tolerance region, the step of performing the coarse alignment includes validating a plurality of alignment data.

12. The method of claim 11, wherein if the validating of the plurality of alignment data results in a validation error, the catheter system (100) is updated, and error handling is initiated, and if the validating of the plurality of alignment data does not result in a validation error, the step of performing the coarse alignment further includes removing a plurality of camera offsets and performing a linear regression with respect to a plurality of hardware offsets.

13. The method of claim 12, wherein the plurality of hardware offsets include at least one of a tilt offset, a y-axis offset, and an x-axis offset, and/or wherein the step of performing the coarse alignment further includes positioning the plurality of hardware based on the plurality of hardware offsets.

14. The method of claim 12 or 13, wherein the step of performing the coarse alignment further includes moving the stage (258, 260) to a third stage location and capturing a plurality of third captured images with the camera (128C) while the stage (258, 260) is in the third stage location.

15. A system controller (126) comprising a processor and a memory, the memory storing instructions that, when executed by the processor, cause the system controller (126) to perform the method of any one of claims 1 to 14.
